# EUROPEAN PATENT APPLICATION

(11) **EP 4 588 525 A1**
(43) Date of publication of application: **23.07.2025**
(21) Application number: 23865523.7
(22) Date of filing: 12.09.2023
(51) Int. Cl.: A61Q 1/00, A61Q 1/06, A61Q 1/10, A61Q 17/04, A61Q 19/00, A61K 8/891

(54) **SILICONE PARTICLES FOR COSMETICS, AND COSMETICS**

(30) Priority: 16.09.2022 JP 2022148398
(71) Applicant: SHIN-ETSU CHEMICAL CO., LTD., Tokyo 1000005 (JP)
(72) Inventor: INOKUCHI Yoshinori, Annaka-shi Gunma 379-0224 (JP); HORIGUCHI Ryuji, Annaka-shi Gunma 379-0224 (JP); TANAKA Mituru, Tokyo 100-0005 (JP)
(74) Representative: Mewburn Ellis LLP
(86) International application number: PCT/JP2023/033197
(87) International publication number: WO 2024/058171

(57) **Abstract**

Silicone particles for cosmetics according to the present invention comprise 100 parts by mass of silicone rubber spherical particles and 0.5-25 parts by mass of a polyorganosilsesquioxane covering the surfaces thereof, and have a volume mean particle diameter of 0.5-100 µm. With respect to 100 parts by mass of said silicone particles for cosmetics, 100 parts by mass or more of ethylhexyl methoxycinnamate is absorbed.

The refractive index of the silicone rubber spherical particles is 1.43-1.51.

## Description

### TECHNICAL FIELD

This invention relates to cosmetic silicone particles having silicone rubber spherical particles covered on their surface with a polyorganosilisesquioxane, and a cosmetic composition comprising the same.

### BACKGROUND ART

From the past, cosmetic compositions having blended therein oily components such as hydrocarbon oils, higher alcohols, ester oils, glyceride oils, natural animal and plant oils and semi-synthetic oils, fluorochemical oils, UV absorbers, non-crosslinked silicone surfactants, other surfactants, silicone oils, modified silicone oils, silicone elastomers, silicone resins and oil-soluble gelling agents are used for the purpose of acquiring skin protecting, softening, smoothening, moisturizing, and other effects. These cosmetic compositions, however, have several drawbacks including heavy to spread, sticky, oily and oil film-forming feelings. For example, Patent Documents 1 and 2 propose cosmetic compositions comprising a crosslinked organopolysiloxane obtained from the addition polymerization of an organohydrogenpolysiloxane with an organopolysiloxane, and an oil. These cosmetic compositions eliminate most of the above-mentioned drawbacks, but still have a problem that in the course of skin application, they become heavy to spread as the oil volatilizes off and subsequently lose dryness.

Make-up cosmetics have a problem that after applied to the skin, cosmetic characteristics alter under the action of sebum secreted out of the skin. That is, the adhesion of the cosmetic to the skin at the site of application is reduced by the sebum, and if brought in contact with the skin at a different site or fabric, the cosmetic is readily transferred to the skin or fabric. Further, the sebum tends to change the color of the cosmetic and makes the cosmetic more sheen.

Patent Document 3 proposes to formulate a sebum-absorbing powder in a make-up cosmetic composition. However, the sebum is kept adsorbed on the surface of particles or absorbed in gaps among particles, but not absorbed in the bulk of particles. Then the amount of sebum absorbed is not so large and the effect of preventing the cosmetic from discoloring and sheening is not sufficient.

On the other hand, silicone particles are used for the purposes of imparting a feeling on use like dryness or smoothness and spreadability to cosmetics. In particular, silicone microparticles in the form of silicone rubber spherical particles covered with polyorganosilsesquioxane (see Patent Document 3) are used in many cosmetics because they give a soft feeling on use and are non-agglomerative and well dispersible.

### PRIOR ART DOCUMENTS

### PATENT DOCUMENTS

Patent Document 1: WO 2003/024413
Patent Document 2: WO 1997/004737
Patent Document 3: JP-A H07-196815

### SUMMARY OF INVENTION

### TECHNICAL PROBLEM

An object of the invention, which has been made under the above-mentioned circumstances, is to provide silicone particles which are used to formulate a cosmetic composition that eliminates drawbacks such as heaviness, stickiness, oiliness and oil film feeling, has smooth spreading, skin penetration, and pleasant feeling on use, prevents make-up cosmetics from altering their characteristics, changing their color, and sheening, and makes rough skin texture (like wrinkles and roughness) less noticeable by taking advantage of light diffusion or gradation effect; and a cosmetic composition comprising the same.

### SOLUTION TO PROBLEM

Making extensive investigations to attain the above object, the inventors have found that cosmetic silicone particles comprising specific amounts of silicone rubber spherical particles and polyorganosilsesquioxane covering their surface and having a specific volume average particle size can solve the outstanding problems when the refractive index and preparation of the silicone rubber spherical particles are specified. The invention is predicated on this finding.

Accordingly, the invention provides cosmetic silicone particles and a cosmetic composition as defined below.
1. Cosmetic silicone particles comprising 100 parts by weight of silicone rubber spherical particles and 0.5 to 25 parts by weight of a polyorganosilsesquioxane covering the surface thereof, and having a volume average particle size of 0.5 to 100 µm,
   100 parts by weight of the cosmetic silicone particles absorb at least 100 parts by weight of ethylhexyl methoxycinnamate,
   the silicone rubber spherical particles have a refractive index of 1.43 to 1.51, and
   the silicone rubber spherical particles are the addition reaction product of

   (A) an organopolysiloxane containing at least two alkenyl groups per molecule, represented by the average compositional formula (1):

      R¹ₐR²_{b}SiO_{(4-a-b)/2} (1)

      wherein R¹ is each independently a substituted or unsubstituted, alkenyl-free, C₁-C₃₀ monovalent hydrocarbon group, 1 to 35 mol% of all R¹ being phenyl, R² is each independently a C₂-C₆ alkenyl group, a and b are numbers in the range: 0 < a < 3, 0 < b < 3, and 0.1 ≤ a+b < 3, and
   (B) an organohydrogenpolysiloxane containing at least two silicon-bonded hydrogen atoms per molecule, represented by the average compositional formula (2):

      R³_{c}H_{d}SiO_{(4-c-d)/2} (2)

      wherein R³ is each independently a substituted or unsubstituted, alkenyl-free, C₁-C₃₀ monovalent hydrocarbon group, 1 to 35 mol% of all R³ being phenyl, c and d are numbers in the range: 0 < c < 3, 0 < d ≤ 3, and 0.1 ≤ c+d ≤ 3,
   excluding the combination of (A) an organopolysiloxane containing only two alkenyl groups per molecule with (B) an organohydrogenpolysiloxane containing only two SiH groups per molecule.
2. The cosmetic silicone particles of 1 wherein 5 to 28 mol% of all R¹ in formula (1) is phenyl, and 5 to 25 mol% of all R³ in formula (2) is phenyl.
3. The cosmetic silicone particles of 1 or 2 wherein 100 parts by weight of the cosmetic silicone particles absorb at least 90 parts by weight of a silicone based oil selected from silicone oils and modified silicone oils and at least 50 parts by weight of a hydrocarbon based oil selected from sebum, hydrocarbon oils, and ester oils.
4. The cosmetic silicone particles of any one of 1 to 3 wherein the silicone rubber spherical particles have a rubber hardness of 20 to 40 as measured by Type A Durometer according to JIS K6253.
5. A cosmetic composition comprising the cosmetic silicone particles of any one of 1 to 4.

### ADVANTAGEOUS EFFECTS OF INVENTION

When the silicone particles are formulated in a cosmetic composition, the resulting cosmetic composition eliminates drawbacks such as heaviness, stickiness, oiliness and oil film feeling, has smooth spreading, skin penetration, and a pleasant feeling on use, prevents make-up cosmetics from altering their characteristics, changing their color, and sheening, and makes rough skin texture (like wrinkles and roughness) less noticeable by taking advantage of light diffusion or gradation effect. According to the invention, there are provided such silicone particles and a cosmetic composition comprising the same.

### BRIEF DESCRIPTION OF DRAWINGS

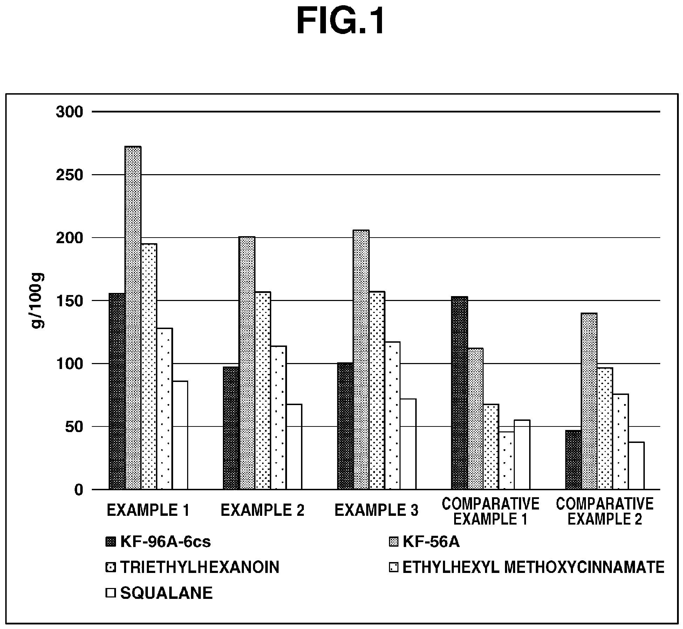

[FIG. 1] FIG. 1 is a graph showing the results of Examples.

### DESCRIPTION OF EMBODIMENTS

Now the invention is described in detail. Herein, the name of ingredients is sometimes expressed by the Japanese labeling name or the International Nomenclature for Cosmetic Ingredients (INCI). When the Japanese labeling name is identical with the INCI, sometimes either one is omitted.

### [Cosmetic silicone particles]

The invention provides cosmetic silicone particles comprising 100 parts by weight of silicone rubber spherical particles and 0.5 to 25 parts by weight of a polyorganosilsesquioxane covering the surface thereof, and having a volume average particle size of 0.5 to 100 µm, wherein 100 parts by weight of the cosmetic silicone particles absorb at least 100 parts by weight of ethylhexyl methoxycinnamate. Hereinafter, "cosmetic silicone particles" is simply referred to as "silicone particles." In the silicone particles having silicone rubber spherical particles covered on their surface with a polyorganosilsesquioxane, the cover may extend over parts or the entirety of silicone rubber spherical particles.

The silicone particles should have a volume average particle size of 0.5 to 100 µm, preferably 1 to 40 µm, more preferably 1 to 10 µm. If the volume average particle size is less than 0.5 µm, there is a weak likelihood of gaining dryness and smoothness. If the volume average particle size exceeds 100 µm, there is a low likelihood of gaining dryness and smoothness, and a gritty feeling may be exerted. The volume average particle size is measured by a Coulter counter according to the electrical resistance method (the same holds true, hereinafter).

The silicone particles absorb at least 100 parts by weight, preferably at least 115 parts by weight, more preferably at least 120 parts by weight of ethylhexyl methoxycinnamate per 100 parts by weight of the silicone particles. The upper limit, which is not critical, may be selected as appropriate from a range of up to 130 parts by weight. If the oil absorption is less than 100 parts by weight of ethylhexyl methoxycinnamate, it is impossible to fully absorb ethylhexyl methoxycinnamate in cosmetics, and the sticky feeling or glitter inherent to ethylhexyl methoxycinnamate is not fully controlled upon application to the skin, leading to a loss of feeling on use. It is noted that the oil absorption is measured according to JIS K 5101-13-1, Part 13: Oil Absorption, Section 1: refined linseed oil method, using ethylhexyl methoxycinnamate instead of refined linseed oil. The oil absorption may be achieved using phenyl-containing materials like components (A) and (B) in the invention.

The oil absorption per 100 parts by weight of the cosmetic silicone particles is preferably:
at least 90 parts by weight of a silicone based oil selected from silicone oils and modified silicone oils and
at least 50 parts by weight of a hydrocarbon based oil selected from sebum, hydrocarbon oils, and ester oils.

If the oil absorption is less than 90 parts by weight of the silicone based oil, there is a risk that a silicone based oil in cosmetics is not fully absorbed, the light diffusion effect is lowered, and the effect of gradation of rough skin texture (like wrinkles and roughness) upon application to the skin is lost. If the oil absorption is less than 50 parts by weight of the hydrocarbon based oil, there is a risk that the sebum secreted out of the skin is not fully absorbed upon application to the skin, and the adhesion of cosmetics to the skin is reduced by the sebum, and if brought in contact with the skin at a different site or fabric, the cosmetic is readily transferred to the skin or fabric. It is noted that the oil absorption is measured according to JIS K 5101-13-1, Part 13: Oil Absorption, Section 1: refined linseed oil method, using the silicone based oil or hydrocarbon based oil instead of refined linseed oil.

The silicone oil or modified silicone oil is used as a starting ingredient for cosmetics. It is an oil having a kinematic viscosity at 25°C of up to 20 mm²/s. Examples of the silicone oil include a cyclic or straight dimethylpolysiloxane having a kinematic viscosity at 25°C of at least 4 mm²/s and a cyclic or straight dimethylpolysiloxane having a kinematic viscosity at 25°C of at least 4 mm²/s, specifically cyclopentasiloxane (INCI), cyclohexasiloxane (INCI), and dimethicone (INCI). It is noted that the kinematic viscosity is measured at 25°C by a capillary viscometer (the same holds true, hereinafter).

The modified silicone oil refers to a straight dimethylpolysiloxane or modified silicone oil having a kinematic viscosity at 25°C of less than 4 mm²/s. Examples thereof include long chain alkyl-modified silicones having a kinematic viscosity at 25°C of 1.5 to 2 mm²/s, such as dimethicone (INCI), trisiloxane (INCI), methyltrimethicone (INCI), ethyl trisiloxane (INCI), and hexyldimethicone (INCI); straight or branched organopolysiloxanes such as phenyltrimethicone (INCI), diphenyldimethicone (INCI), diphenylsiloxyphenyltrimethicone (INCI), and tetraphenyldimethyldisiloane (INCI); aromatic group-modified silicones such as cyclic organopolysiloxanes, e.g., tetramethyltetraphenyl cyclotetrasiloxane; amino-modified organopolysiloxanes such as amodimethicone (INCI) and aminopropyldimethicone (INCI); pyrrolidone-modified organopolysiloxanes such as PCA dimethicone (INCI); pyrrolidone carboxylic acid-modified organopolysiloxanes, amino acid-modified silicones, and fluorine-modified silicones, with the aromatic group-modified silicones being preferred.

The hydrocarbon oil or ester oil is used as a starting ingredient for cosmetics. Suitable hydrocarbon oils include straight or branched hydrocarbon oils and may be either volatile or non-volatile hydrocarbon oils. Examples include olefin oligomer (INCI), isoparaffins such as (C13, 14) isoparaffin (INCI), isododecane (INCI), undecane (INCI), dodecane (INCI), isohexadecane (INCI), hydrogenated polyisobutene (labeling name, INCI: Hydrogenated Polyisobutene), squalane (INCI), mineral oil (INCI), and alkanes such as coconut alkane (INCI) and (C13-15) alkane (INCI).

Examples of the ester oil include diisobutyl adipate (labeling name, INCI: Diisobutyl Adipate), dihexyldecyl adipate (labeling name), diheptylundecyl adipate (labeling name, INCI: Diheptylundecyl Adipate), n-alkyl glycol monoisostearates such as isostearyl isostearate (labeling name, INCI: Isostearyl Isostearate), isocetyl isostearate (labeling name, INCI: Isocetyl Isostearate), trimethylolpropane triisostearate (labeling name, INCI: Trimethylolpropane Triisostearate), glycol diethylhaxanoate (labeling name, INCI: Glycol Diethylhexanoate), cetyl ethylhexanoate (labeling name, INCI: Cetyl Ethylhexanoate), trimethylolpropane triethylhexanoate (labeling name, INCI: Trimethylolpropane Triethylhexanoate), pentaerythrityl tetraethylhexanoate (labeling name, INCI: Pentaerythrityl Tetraethylhexanoate), cetyl octanoate (labeling name, INCI: Cetyl Ethylhexanoate), octyldodecyl esters such as octyldodecyl stearoyl stearate (labeling name, INCI: Octyldodecyl Stearoyl Stearate), oleyl oleate (labeling name, INCI: Oleyl Oleate), octyldodecyl oleate (labeling name, INCI: Octyldodecyl Oleate), decyl oleate (labeling name, INCI: Decyl Oleate), neopentyl glycol dioctanoate (labeling name, INCI: Neopentyl Glycol Diethylhexanoate), neopentyl glycol dicaprate (labeling name, INCI: Neopentyl Glycol Dicaprate), diisostearyl malate (labeling name, INCI: Diisostearyl Malate), triethyl citrate (labeling name, INCI: Triethyl Citrate), diethylhexyl succinate (labeling name, INCI: Diethylhexyl Succinate), amyl acetate (labeling name, INCI: Amyl Acetate), ethyl acetate (labeling name, INCI: Ethyl Acetate), butyl acetate (labeling name, INCI: Butyl Acetate), isocetyl stearate (labeling name, INCI: Isocetyl Stearate), butyl stearate (labeling name, INCI: Butyl Stearate), diisopropyl sebacate (labeling name, INCI: Diisopropyl Sebacate), diethylhexyl sebacate (labeling name, INCI: Diethylhexyl Sebacate), cetyl lactate (labeling name, INCI: Cetyl Lactate), myristyl lactate (labeling name, INCI: Myristyl Lactate), isononyl isononanoate (labeling name, INCI: Isononyl Isononanoate), isotridecyl isononanoate (labeling name, INCI: Isotridecyl Isononanoate), palmitic acid esters such as isopropyl palmitate (labeling name, INCI: Isopropyl Palmitate), ethylhexyl palmitate (labeling name, INCI: Ethylhexyl Isopalmitate), and hexyldecyl palmitate (labeling name, INCI: Isocetyl Palmitate, Hexyldecyl Palmitate), cholesteryl hydroxystearate (labeling name, INCI: Cholesteryl Hydroxystearate), myristic acid esters such as isopropyl myristate (labeling name, INCI: Isopropyl Myristate), octyldodecyl myristate (labeling name, INCI: Octyldodecyl Myristate), and myristyl myristate (labeling name, INCI: Myristyl Myristate), ethylhexyl laurate (labeling name, INCI: Ethylhexyl Laurate), hexyl laurate (labeling name, INCI: Hexyl Laurate), dioctyldodecyl lauroyl glutamate (labeling name, INCI: Dioctyldodecyl Lauroyl Glutamate), and isopropyl lauroyl sarcosinate (labeling name, INCI: Isopropyl Lauroyl Sarcosinate).

The ester oils include glyceride oils such as triethylhexanoin (INCI), glyceryl tri(caprylate/caprate) (labeling name, INCI: Caprylic/Capric Triglyceride), cocoglyceryl (INCI), caprylic/capric/succinic triglyceride (labeling name, INCI: Caprylic/Capric/Succinic Triglyceride), and caprylic/capric glycerides (labeling name, INCI: Caprylic/Capric Glycerides).

### [Silicone rubber spherical particles]

The silicone particles of the invention are composed of silicone rubber spherical particles covered on their surface with polyorganosilsesquioxane, wherein the shape of silicone rubber spherical particles is spherical. The spherical shape is preferred from the aspect of feeling on use. As used herein, the term "spherical" means that the shape of microparticles encompasses not only a true sphere, but also a deformed sphere having an aspect ratio (=length/breadth) which is on the average in a range from 1.0 to 4.0, preferably from 1.0 to 2.0, more preferably from 1.0 to 1.6, even more preferably from 1.0 to 1.4. The shape of rubber particles can be ascertained by observing under an optical microscope or electron microscope.

The silicone rubber spherical particles have a refractive index of 1.43 to 1.51, and as viewed from the aspect of imparting an appropriate gradation effect to the cosmetic composition, preferably 1.44 to 1.50, more preferably 1.44 to 1.49. There are various types of roughness (or ridges and recesses) on the skin. Fine and regular roughness is essential for the beautiful skin. Deep and irregular roughness of large size like pores and wrinkles is a drawback of the skin. Pores or dips look noticeable for the reason that flat areas (or crista cutis) look bright upon incidence of light, whereas recesses like pores and wrinkles where incident light is not reflected become shadow. A difference in brightness is then produced therebetween. The difference in brightness is exaggerated at large and irregular roughness sites, which become more noticeable. Such roughness is attributable to the morphology of the skin. Even when a high refractive index pigment like titanium dioxide is uniformly coated, the roughness cannot be hided in contrast to color unevenness and the brightness difference is rather exaggerated in some cases. In hiding roughness, the brightness difference between ridges and recesses must be minimized by light diffusion or reflection suppression, as typified by the soft focus theory. This is known as the gradation effect and expressed as gradation in the evaluation in Examples. It is noted that the refractive index is measured on the bulk form of the same composition as the particles. The refractive index of silicone rubber can be adjusted by the phenyl contents of components (A) and (B). The higher the phenyl content, the higher becomes the refractive index. There is also a tendency that the greater the amount of alkenyl groups in component (A) or the greater the amount of silicon-bonded hydrogen atoms in component (B), the higher becomes the refractive index, although a greater change cannot be achieved.

The silicone rubber of which silicone rubber spherical particles are composed is preferably free of stickiness and also preferably has a rubber hardness in the range of 20 to 40 as measured by Type A Durometer according to JIS K-6253. If the rubber hardness is too high, there is a risk that silicone rubber spherical particles themselves are hard. Then slipperiness on the skin is improved, but smoothness is lost. Also the elasticity of silicone rubber spherical particles is lost. If the rubber hardness is too low, silicone rubber spherical particles themselves are soft. Then smoothness on the skin is improved, but slipperiness is lost. Also the elasticity of silicone rubber spherical particles themselves is improved, but the spread after applied to the skin is heavily loaded, and extension or spread is aggravated, leaving some accumulation as creases. In order to impart appropriate slipperiness and smoothness to the cosmetic composition, the rubber hardness is preferably 20 to 40, more preferably 23 to 38, even more preferably 25 to 35. As used herein, the rubber hardness is obtained by measuring a sample which is formed from the composition of particles to the shape and size according to JIS K-6253.

The silicone rubber spherical particles should preferably have a volume average particle size of 0.5 to 100 µm, more preferably 1 to 40 µm, more preferably 1 to 10 µm.

The silicone rubber spherical particles are the addition reaction product of
(A) an organopolysiloxane containing at least two alkenyl groups per molecule, represented by the average compositional formula (1):

   R¹ₐR²_{b}SiO_{(4-a-b)/2} (1)

   wherein R¹ is each independently a substituted or unsubstituted, alkenyl-free, C₁-C₃₀ monovalent hydrocarbon group, 1 to 35 mol% of all R¹ being phenyl, R² is each independently a C₂-C₆ alkenyl group, a and b are numbers in the range: 0 < a < 3, 0 < b < 3, and 0.1 ≤ a+b < 3, and
(B) an organohydrogenpolysiloxane containing at least two silicon-bonded hydrogen atoms per molecule, represented by the average compositional formula (2):

   R³_{c}H_{d}SiO_{(4-c-d)/2} (2)

   wherein R³ is each independently a substituted or unsubstituted, alkenyl-free, C₁-C₃₀ monovalent hydrocarbon group, 1 to 35 mol% of all R³ being phenyl, c and d are numbers in the range: 0 < c < 3, 0 < d ≤ 3, and 0.1 ≤ c+d ≤ 3,
excluding the combination of (A) an organopolysiloxane containing only two alkenyl groups per molecule with (B) an organohydrogenpolysiloxane containing only two SiH groups per molecule.

Component (A) is an organopolysiloxane containing at least two alkenyl groups per molecule, represented by the average compositional formula (1):

R¹ₐR²_{b}SiO_{(4-a-b)/2} (1)

wherein R¹ is each independently a substituted or unsubstituted, alkenyl-free, C₁-C₃₀ monovalent hydrocarbon group, 1 to 35 mol% of all R¹ being phenyl, R² is each independently a C₂-C₆ alkenyl group, a and b are numbers in the range: 0 < a < 3, 0 < b < 3, and 0.1 ≤ a+b < 3, which may be used alone or in admixture of two or more.

R¹ is each independently a substituted or unsubstituted, alkenyl-free, C₁-C₃₀ monovalent hydrocarbon group, some R¹ being phenyl. R¹ other than phenyl is each independently a substituted or unsubstituted, alkenyl-free, C₁-C₃₀, preferably C₁-C₂₂, more preferably C₁-C₁₈ monovalent hydrocarbon group. Examples of R¹ other than phenyl include alkyl groups such as methyl, ethyl, propyl, butyl, pentyl, hexyl, heptyl, octyl, decyl, undecyl, dodecyl, tetradecyl, pentadecyl, hexadecyl, heptadecyl, octadecyl, nonadecyl, icosyl, henicosyl, docosyl, tricosyl, tetracosyl, and triacontyl; aryl groups such as phenyl, tolyl and naphthyl; aralkyl groups such as benzyl and phenethyl; cycloalkyl groups such as cyclopentyl, cyclohexyl, and cycloheptyl; and the foregoing hydrocarbon groups in which some or all of the carbon-bonded hydrogen atoms are substituted by atoms such as halogen atoms (e.g., fluorine, chlorine, bromine and iodine) and/or substituents such as acryloyloxy, methacryloyloxy, epoxy, glycidoxy, and carboxyl. Phenyl accounts for 1 to 35 mol%, preferably 5 to 28 mol%, more preferably 5 to 25 mol%, even more preferably 10 to 25 mol% of all R¹. Preferably methyl accounts for at least 50 mol%, more preferably at least 70 mol% of R¹ other than phenyl. As used herein, "mol%" is defined as a proportion of the number (moles) of each monovalent hydrocarbon group to the total number (total moles) of monovalent hydrocarbon groups in R¹.

R² is each independently a C₂-C₆ alkenyl group, for example, vinyl, allyl, propenyl, butenyl, pentenyl, and hexenyl, preferably vinyl.

The subscripts a and b are numbers in the range: 0 < a < 3, 0 < b < 3, and 0.1 ≤ a+b < 3, and preferably positive numbers in the range: 0 < a ≤ 2.295, 0.005 ≤ b ≤ 2.3, and 0.5 ≤ a+b ≤ 2.3.

Component (A) has a viscosity at 25°C of preferably up to 100,000 mm²/s, more preferably up to 10,000 mm²/s, even more preferably up to 5,000 mm²/s. When the viscosity is up to 100,000 mm²/s, it is quite easy to prepare silicone microparticles having a narrow particle size distribution by the preparation method shown later. The lower limit of viscosity may be at least 0.7 mm²/s, preferably at least 2 mm²/s, though not critical. Also, component (A) may have a linear, cyclic or branched structure, preferably a linear structure or branched structure containing less branched units. The point of attachment of alkenyl group is not particularly limited, that is, the alkenyl group may be attached to any silicon atom at a side chain or end of the molecule. Most preferably, the alkenyl groups are attached to the silicon atoms at the both ends of the linear organopolysiloxane.

Exemplary of the linear organopolysiloxane is a structure having the general formula (3). The order of arrangement of siloxane units is not limited to the illustrated one (the same holds true, hereinafter).

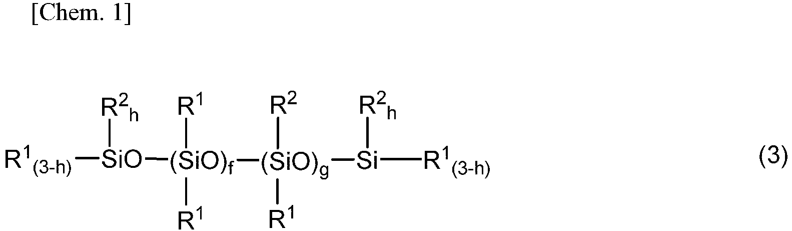

Herein R¹ and R² are as defined above, f is a positive number, g is 0 or a positive number, and h is 0, 1, 2 or 3, with the proviso that g and h are numbers satisfying g+2×h ≥ 2.

Exemplary of the branched organopolysiloxane is a structure which is branched from R²SiO_{3/2} unit, having the general formula (4).

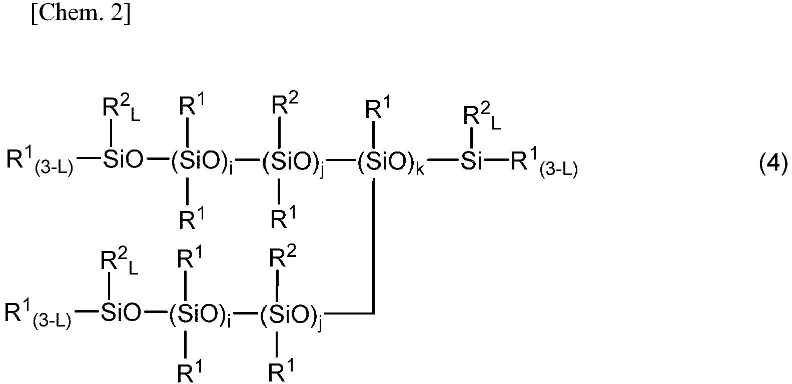

Herein R¹ and R² are as defined above, i is a positive number, j is 0 or a positive number, k is a positive number, and L is 0, 1, 2, or 3, with the proviso that j and L are numbers satisfying j+L ≥ 1.

Exemplary of the branched organopolysiloxane is a structure which is branched from SiO_{4/2} unit, having the general formula (5).

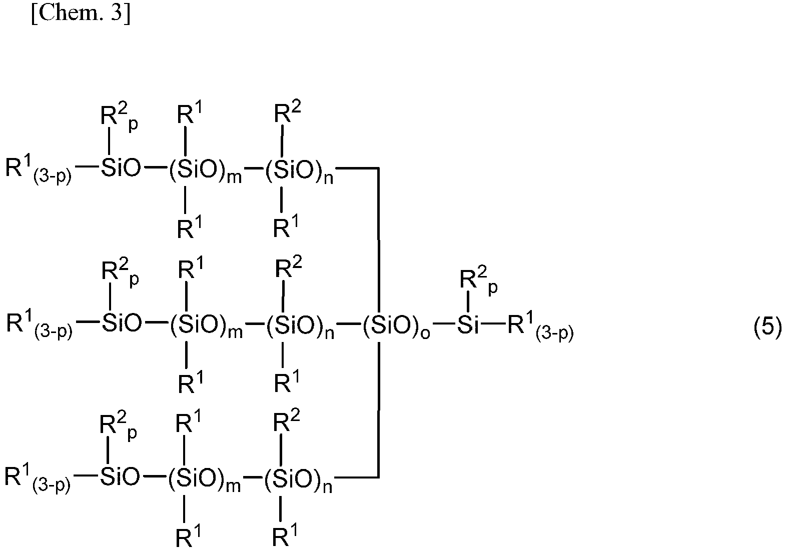

Herein R¹ and R² are as defined above, m is a positive number, n is 0 or a positive number, o is a positive number, and p is 0, 1, 2, or 3, with the proviso that n and p are numbers satisfying n+p ≥ 1.

Also exemplary is an organopolysiloxane having at least two alkenyl groups per molecule, represented by the unit formula (6).

[R¹₃SiO_{1/2}]_{q}[R²(R¹)₂SiO_{1/3}]ᵣ[SiO_{4/2}]ₛ (6)

Herein R¹ and R² are as defined above, q is 0 or a positive number, r is a positive number, and s is a positive number.

Component (B) is an organohydrogen-polysiloxane containing at least two silicon-bonded hydrogen atoms per molecule, represented by the average compositional formula (2):

R³_{c}H_{d}SiO_{(4-c-d)/2} (2)

wherein R³ is each independently a substituted or unsubstituted, alkenyl-free, C₁-C₃₀ monovalent hydrocarbon group, 1 to 35 mol% of all R³ being phenyl, c and d are numbers in the range: 0 < c < 3, 0 < d ≤ 3, and 0.1 ≤ c+d ≤ 3, which may be used alone or in admixture of two or more.

R³ is each independently a substituted or unsubstituted, alkenyl-free, C₁-C₃₀ monovalent hydrocarbon group, some R³ being phenyl. R³ other than phenyl is preferably a C₁-C₂₂, more preferably C₁-C₁₈ monovalent hydrocarbon group. Examples of R³ other than phenyl are as exemplified above in conjunction with formula (1) as component (A). Phenyl accounts for 1 to 35 mol%, preferably 5 to 25 mol% of all R³. Preferably methyl accounts for at least 80 mol%, more preferably at least 95 mol% of R³ other than phenyl. As used herein, "mol%" is defined as a proportion of the number (moles) of each monovalent hydrocarbon group to the total number (total moles) of monovalent hydrocarbon groups in R³.

The subscripts c and d are numbers in the range: 0 < c < 3, 0 < d ≤ 3, and 0.1 ≤ c+d < 3, and preferably positive numbers in the range: 0 < c ≤ 2.295, 0.005 ≤ d ≤ 2.3, and 0.5 ≤ c+d ≤ 2.3.

Component (B) has a viscosity at 25°C of preferably up to 100,000 mm²/s, more preferably up to 10,000 mm²/s. When the viscosity is up to 100,000 mm²/s, it is quite easy to prepare silicone microparticles having a narrow particle size distribution by the preparation method shown later. The lower limit of viscosity may be at least 0.4 mm²/s, preferably at least 2 mm²/s, though not critical. Also, component (B) may have a linear, cyclic or branched structure, preferably a linear or branched structure. The position of attachment of silicon-bonded hydrogen is not particularly limited, that is, hydrogen may be attached to any silicon atom at a side chain or end of the molecule.

Exemplary of the linear organopolysiloxane (B) is one having the general formula (7).

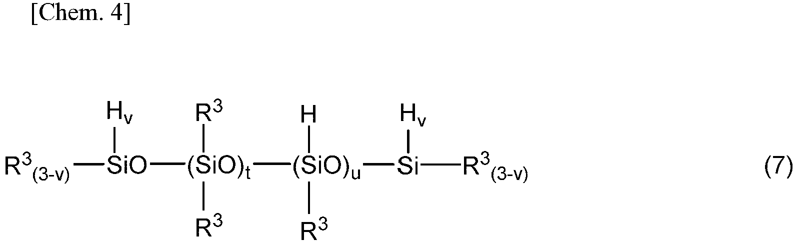

Herein R³ is as defined above, t is a positive number, u is 0 or a positive number, and v is 0, 1, 2 or 3, with the proviso that u and v are numbers satisfying u+2×v ≥ 2.

Exemplary of the branched structure is one which is branched from R³SiO_{3/2} unit, having the general formula (8).

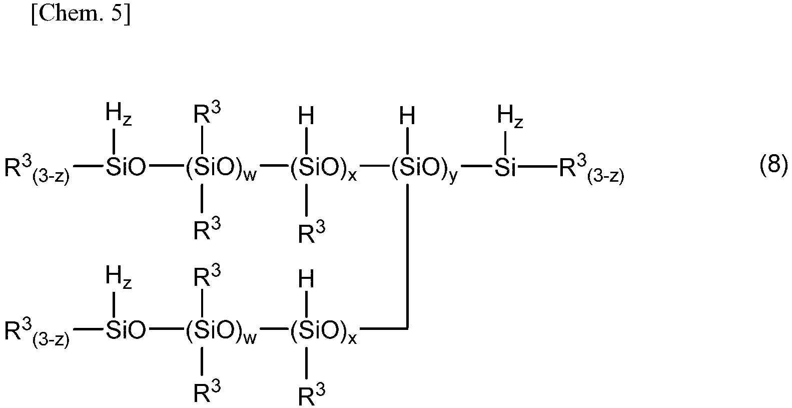

Herein R³ is as defined above, w is a positive number, x is 0 or a positive number, y is a positive number, and z is 0, 1, 2, or 3, with the proviso that x and z are numbers satisfying x+z ≥ 1.

Exemplary of the branched structure is one which is branched from SiO_{4/2} unit, having the general formula (9).

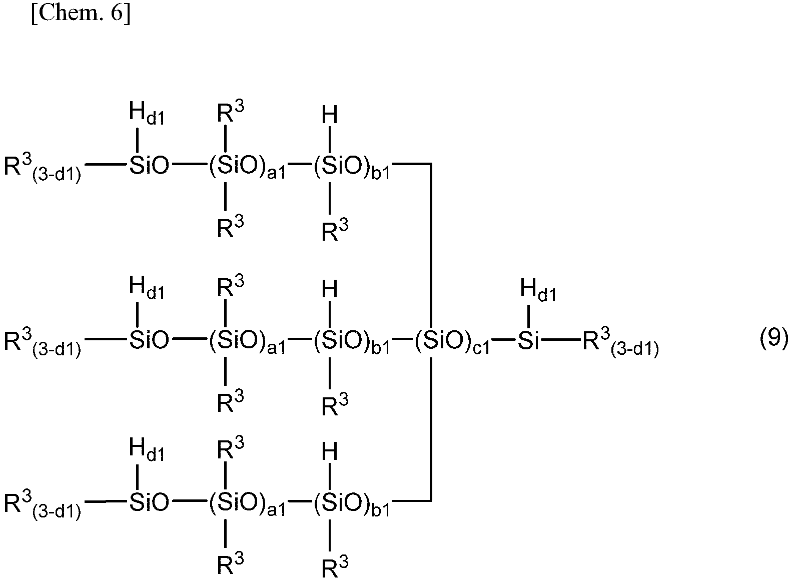

Herein R³ is as defined above, a1 is a positive number, b1 is 0 or a positive number, c1 is a positive number, and d1 is 0, 1, 2, or 3, with the proviso that b1 and d1 are numbers satisfying b1+d1 ≥ 1.

Also exemplary is a siloxane having at least two alkenyl groups per molecule, represented by the unit formula (10).

[R³₃SiO_{1/2}]ₑ₁[H(R³)₂SiO_{1/3}]_{f1}[SiO_{4/2}]_{g1} (10)

Herein R³ is as defined above, e1 is 0 or a positive number, f1 is a positive number, and g1 is a positive number.

As mentioned above, component (A) is an organopolysiloxane containing at least two alkenyl groups per molecule and component (B) is an organohydrogenpolysiloxane containing at least two silicon-bonded hydrogen atoms per molecule, with the proviso that the combination of (A) an organopolysiloxane containing only two alkenyl groups per molecule with (B) an organohydrogenpolysiloxane containing only two SiH groups per molecule is excluded. Differently stated, when component (A) has two alkenyl groups, at least one of component (B) is an organohydrogensiloxane having at least three SiH groups. When component (B) has two SiH groups, at least one of component (A) is an organosiloxane having at least three alkenyl groups.

The silicone rubber spherical particles are the addition reaction product of components (A) and (B). The amount of component (B) relative to component (A) is such that the ratio of the number of SiH groups in component (B) to the number of monovalent alkenyl groups in component (A) may range preferably from 0.5 to 2.0, more preferably from 0.7 to 1.5. If component (B) is blended in such amounts that the number ratio of SiH groups may be less than the lower limit or more than the upper limit, the resulting silicone rubber cured product may become sticky or have too high reaction activity.

Typical of the addition reaction catalyst are platinum group metal base catalysts for use in hydrosilation reaction. Examples include platinum group metal elements such as platinum (inclusive of platinum black), rhodium and palladium; platinum chloride, chloroplatinic acid and chloroplatinic acid salts such as H₂PtCl₄·xH₂O, H₂PtCl₆·xH₂O, NaHPtCl₆·xH₂O, KHPtCl₆·xH₂O, Na₂PtCl₆·xH₂O, K₂PtCl₄·xH₂O, PtCl₄·xH₂O, PtCl₂, and Na₂HPtCl₄·xH₂O wherein x is an integer of 0 to 6, preferably 0 or 6; alcohol-modified chloroplatinic acids; complexes of platinum chloride and chloroplatinic acid with olefins; complexes of chloroplatinic acid with vinyl-bearing siloxanes, complexes of platinum with vinyl-bearing siloxanes; platinum group metals (e.g., platinum black and palladium) on supports (e.g., alumina, silica, carbon); rhodium-olefin complexes, and chlorotris(triphenylphosphine)rhodium, known as Wilkinson catalyst. These may be used alone or in admixture.

The amount of the platinum group metal base catalyst used is an amount effective as the hydrosilation reaction catalyst. The amount of platinum group metal in the platinum group metal base catalyst, calculated on a weight basis, is typically about 0.1 to about 500 ppm, preferably 0.5 to 200 ppm, more preferably 0.1 to 100 ppm based on the total weight of components (A) and (B).

The silicone rubber spherical particles may contain a silicone oil, organosilane, inorganic powder, organic powder, and antioxidant.

### [Polyorganosilsesquioxane]

In the silicone particles of the invention, the silicone rubber spherical particles are covered on their surface with a polyorganosilsesquioxane, which is a polymer comprising units having the formula (11):

R⁴SiO_{3/2} (11)

wherein R⁴ is a substituted or unsubstituted C₁-C₂₀ monovalent hydrocarbon group. The polymer may contain alkoxy groups originating from an organotrialkoxysilane used as a starting material and silanol groups left unreacted after condensation reaction.

R⁴ is a substituted or unsubstituted C₁-C₂₀, preferably C₁-C₆ monovalent hydrocarbon group. Examples of R⁴ include alkyl groups such as methyl, ethyl, propyl, butyl, pentyl, hexyl, heptyl, octyl, decyl, undecyl, dodecyl, tetradecyl, pentadecyl, hexadecyl, heptadecyl, octadecyl, nonadecyl, icosyl, henicosyl, docosyl, tricosyl, and tetracosyl; aryl groups such as phenyl, tolyl and naphthyl; aralkyl groups such as benzyl and phenethyl; alkenyl groups such as vinyl and allyl; cycloalkyl groups such as cyclopentyl, cyclohexyl, and cycloheptyl; and the foregoing hydrocarbon groups in which some or all of the carbon-bonded hydrogen atoms are substituted by atoms such as halogen atoms (e.g., fluorine, chlorine, bromine and iodine) and/or substituents such as amino, acryloyloxy, methacryloyloxy, epoxy, glycidoxy, and carboxyl. Preferably methyl accounts for at least 50 mol%, more preferably at least 80 mol% of all R⁴. As used herein, "mol%" is defined as a proportion of the number (moles) of each monovalent hydrocarbon group to the total number (total moles) of monovalent hydrocarbon groups in R⁴.

In addition to the R⁴SiO_{3/2} units, the polyorganosilsesquioxane may contain one or more of R⁴₂SiO_{2/2} units, R⁴₃SiO_{1/2} units, and SiO_{4/2} units as long as the properties of the resulting silicone particles including feeling on use (dryness and smoothness), soft feeling, non-agglomeration, and dispersion are not impaired. In the polyorganosilsesquioxane, the content of R⁴SiO_{3/2} units is preferably 70 to 100 mol%, more preferably 80 to 100 mol% of the overall siloxane units.

In the silicone particles, the covering polyorganosilsesquioxane preferably takes the shape of grains having a grain size of up to 500 nm. The polyorganosilsesquioxane may cover parts or the entirety of the surface of silicone rubber spherical particles, preferably cover the overall surface of silicone rubber spherical particles without substantially leaving gaps. The coverage state and grain size can be ascertained by observing the particle surface under an electron microscope.

The covering amount of the polyorganosilsesquioxane is 0.5 to 25 parts by weight, preferably 1 to 15 parts by weight per 100 parts by weight of the silicone rubber spherical particles. If the amount of the polyorganosilsesquioxane is less than 0.5 part by weight, the resulting silicone particles are likely to agglomerate together and lack fluidity, dispersibility, dry feeling and smoothness. If the amount exceeds 25 parts by weight, the resulting silicone particles lack soft touch.

### [Method for preparing silicone particles]

Covering the surface of particles with another material belongs to the technology of forming composite particles and encompasses a variety of techniques. For example, there are known a method of dry mixing particles serving as a core (referred to as "core particles," hereinafter) and particles covering the surface thereof (referred to as "covering particles," hereinafter) for letting the covering particles adhere to the surface of core particles, and a method of further carrying out treatment on the covered particles to apply impact force, compression force, frictional force or shear force for thereby fixedly securing the covering particles to the surface of core particles to form a cover film. However, since the silicone rubber particles are strongly agglomerative, it is difficult to let the covering particles adhere to the surface of silicone rubber particles thinly and uniformly. Also, since the silicone rubber particles are elastic, it is impossible to fixedly secure the covering particles to the surface of silicone rubber particles by applying the impact force, compression force, frictional force or shear force. It is also known to prepare covered particles by spray drying a liquid dispersion of core particles and covering particles. Then, agglomerated particles, particles consisting of covering particles and both will be created.

Then, the silicone particles are preferably prepared by the method described in Patent Document 1. That is, the silicone particles are preferably prepared by subjecting an organotrialkoxysilane to hydrolytic condensation in a water medium in the presence of silicone rubber spherical particles and an alkaline substance, for thereby covering the surface of silicone rubber spherical particles with a polyorganosilsesquioxane.

The silicone rubber spherical particles may be prepared in the form of a water dispersion by a well-known method. That is, a surfactant and water are added to the aforementioned curable liquid silicone composition, and the mixture is emulsified into an oil-in-water emulsion, followed by curing reaction. With this method of emulsifying the curable liquid silicone composition, followed by curing reaction, there are obtained particles of spherical shape.

When silicone rubber spherical particles are obtained from curing by addition reaction, one typical method is by adding a surfactant and water to a curable liquid silicone composition consisting of the alkenyl-containing organopolysiloxane and the organohydrogenpolysiloxane, emulsifying the mixture into an emulsion, adding a platinum group metal based catalyst thereto, and effecting addition reaction.

The surfactant used herein is not particularly limited and may be selected from nonionic surfactants, anionic surfactants, cationic surfactants, and ampholytic surfactants. The surfactants may be used alone or in admixture of two or more.

Examples of the nonionic surfactant include polyoxyethylene alkyl ethers, polyoxyethylene polyoxypropylene alkyl ethers, polyoxyethylene alkyl phenyl ethers, polyethylene glycol fatty acid esters, sorbitan fatty acid esters, polyoxyethylene sorbitan fatty acid esters, polyoxyethylene sorbitol fatty acid esters, glycerol fatty acid esters, polyoxyethylene glycerol fatty acid esters, polyglycerol fatty acid esters, propylene glycol fatty acid esters, polyoxyethylene castor oil, polyoxyethylene hardened castor oil, polyoxyethylene hardened castor oil fatty acid esters, polyoxyethylene alkyl amines, polyoxyethylene fatty acid amines, polyoxyethylene-modified organopolysiloxanes, and polyoxyethylene polyoxypropylene-modified organopolysiloxanes.

Examples of the anionic surfactant include alkyl sulfuric acid ester salts such as sodium laurylsulfate, polyoxyethylene alkyl ether sulfuric acid ester salts, polyoxyethylene alkyl phenyl ether sulfuric acid ester salts, sulfuric acid ester salts of fatty acid alkylol amides, alkylbenzenesulfonic acid salts, polyoxyethylene alkyl phenyl ether sulfonic acid salts, α-olefin sulfonic acid salts, α-sulfofatty acid ester salts, alkylnaphthalene sulfonic acid salts, alkyl diphenyl ether disulfonic acid salts, alkane sulfonic acid salts, N-acyltauric acid salts, dialkylsulfosuccinic acid salts, monoalkylsulfosuccinic acid salts, polyoxyethylene alkyl ether sulfosuccinic acid salts, fatty acid salts, polyoxyethylene alkyl ether carboxylic acid salts, N-acylamino acid salts, monoalkyl phosphoric acid ester salts, dialkyl phosphoric acid ester salts, and polyoxyethylene alkyl ether phosphoric acid ester salts.

Examples of the cationic surfactant include alkyltrimethylammonium salts, dialkyldimethylammonium salts, polyoxyethylene alkyl dimethylammonium salts, dipolyoxyethylene alkylmethylammonium salts, tripolyoxyethylene alkylammonium salts, alkylbenzyldimethylammonium salts, alkylpyridinium salts, monoalkylamine salts, and monoalkylamide amine salts.

Examples of the ampholytic surfactants include alkyldimethylamine oxides, alkyldimethylcarboxybetaine, alkylamidopropyldimethylcarboxybetaine, alkylhydroxysulfobetaine, and alkylcarboxymethyl hydroxyethyl imidazolinium betaine.

Of the foregoing surfactants, the nonionic surfactants are preferred because a curable liquid silicone composition can be emulsified with a small amount and fine particles are obtainable. From the aspect of ease of coverage, the amount of the surfactant used is preferably up to 20 parts by weight per 100 parts by weight of the curable liquid silicone composition. From the aspect of obtaining fine particles, the amount of the surfactant used is preferably at least 0.01 part by weight, more preferably 0.01 to 20 parts by weight, even more preferably 0.05 to 5 parts by weight.

Emulsification may be performed by ordinary emulsifying/dispersing machines. Examples include high-speed rotation centrifugal agitators such as Homodisper, high-speed rotation shear agitators such as Homomixer, high-pressure jet emulsifying/dispersing machines such as Homogenizer, colloid mills, and ultrasonic emulsifiers. The speed and time of agitation are not particularly limited as long as the desired particle size is obtainable.

Once the emulsion is formed, a platinum group metal based catalyst is added thereto. When the catalyst is less dispersible in water, it is preferably dissolved with the aid of a surfactant before it is added to the emulsion. The surfactant is selected from the foregoing surfactants, with the nonionic surfactants being preferred.

Another method of previously adding a platinum group metal based catalyst to the curable liquid silicone composition is acceptable. In this case, it is necessary to retard the reaction by adjusting the temperature, adjusting the amount of the catalyst, or blending a reaction inhibitor, until the end of emulsification.

The addition reaction may be carried out at room temperature (1 to 30°C) or if the reaction is not completed, by heating at a temperature of less than 100°C. The reaction time is selected as appropriate.

From the afore-mentioned method, a water dispersion of silicone rubber spherical particles is obtained. In the step of adding an organotrialkoxysilane to effect hydrolytic condensation, the amount of silicone rubber spherical particles is preferably 1 to 150 parts by weight, more preferably 3 to 70 parts by weight per 100 parts by weight of water as the dispersing medium. Emulsification is carried out such that the desired amount of silicone rubber spherical particles may be obtained. When the amount of silicone rubber spherical particles is excessive, ajustment may be made by diluting with water.

The alkaline substance acts as a catalyst for hydrolytic condensation reaction of an organotrialkoxysilane. The alkaline substance may be used alone or in admixture.

The alkaline substance is not particularly limited. Examples include alkali metal hydroxides such as potassium hydroxide, sodium hydroxide and lithium hydroxide; alkaline earth metal hydroxides such as calcium hydroxide and barium hydroxide; alkali metal carbonates such as potassium carbonate and sodium carbonate; ammonia; tetraalkylammonium hydroxides such as tetramethylammonium hydroxide and tetraethylammonium hydroxide; and amines such as monomethylamine, monoethylamine, monopropylamine, monobutylamine, monopentylamine, dimethylamine, diethylamine, trimethylamine, triethanolamine, and ethylenediamine. These may be used alone or in admixture of two or more. Inter alia, ammonia is most preferred because it can be readily volatilized off from the powder of silica-coated particles. The ammonia used herein may be commercially available ammonia water.

The alkaline substance may be added as such or as an alkaline aqueous solution. The amount of alkaline substance added is such that a water dispersion of silicone rubber spherical microparticles containing the alkaline substance may have a pH value of preferably 10.0 to 13.0, more preferably 10.5 to 12.5. When the alkaline substance is added in such an amount as to give a pH value of 10.0 to 13.0, the hydrolytic condensation reaction of the organotrialkoxysilane and the coverage of the surface of silicone rubber spherical particles with the polyorganosilsesquioxane take place to a sufficient extent.

The organotrialkoxysilane is a starting substance to the polyorganosilsesquioxane. It undergoes hydrolytic condensation under the catalysis of an alkaline substance into a polyorganosilsesquioxane. Exemplary of the organotrialkoxysilane is one having the formula (12):

R⁴Si(OR⁵)₃ (12)

wherein R⁴ is as defined above, and R⁵ is an unsubstituted C₁-C₆ monovalent hydrocarbon group. Examples of the monovalent hydrocarbon group R⁵ include methyl, ethyl, propyl, butyl, pentyl and hexyl, with methyl being preferred from the aspect of reactivity.

When it is desired to incorporate at least one of R⁴₂SiO_{2/2} units, R⁴₃SiO_{1/2} units and SiO_{4/2} units into the polyorganosilsesquioxane, at least one of R⁴₂Si(OR⁵)₂, R⁴₃SiOR⁵ and Si(OR⁵)₄ corresponding these units may be added.

The amount of the organotrialkoxysilane added is set so as to provide 0.5 to 25 parts by weight, preferably 1 to 15 parts by weight of the covering polyorganosilsesquioxane per 100 parts by weight of silicone rubber spherical microparticles.

The organotrialkoxysilane may be added while stirring with a standard agitator such as a propeller, plate or other impeller. The organotrialkoxysilane may be added all at once, but preferably gradually over a time. The temperature during addition is preferably 0 to 60°C, more preferably 0 to 40°C.

While the organotrialkoxysilane is added, at least one selected from cationic surfactants and cationic water-soluble polymers may be added. Such an additive may be added prior to or simultaneously with the addition of the organotrialkoxysilane.

Examples of the cationic surfactant include those exemplified above in the method of preparing the silicone rubber spherical particles. Examples of the cationic water-soluble polymer include polymers of dimethyldiallylammonium chloride, polymers of vinylimidazoline, polymers of methylvinylimidazolium chloride, polymers of acryloxyethyltrimethylammonium chloride, polymers of methacryloxyethyltrimethylammonium chloride, polymers of acrylamidopropyltrimethylammonium chloride, polymers of methacrylamidopropyltrimethylammonium chloride, epichlorohydrin/dimethylamine polymers, ethyleneimine polymers, quaternized forms of ethyleneimine polymers, allylamine hydrochloride polymers, polylysin, cationic starch, cationic cellulose, chitosan, and derivatives of the foregoing having copolymerized therewith monomers having a nonionic or anionic group. The polyorganosilsesquioxane thus formed deposits on the surface of silicone rubber spherical particles. The silicone rubber spherical particles are then covered on their surface with the polyorganosilsesquioxane.

After addition of the organotrialkoxysilane, the agitation is continued until hydrolytic condensation of the organotrialkoxysilane is completed. The reaction may be performed at room temperature or at an elevated temperature of about 40 to about 100°C in order to bring hydrolytic condensation to completion.

At the end of hydrolytic condensation, water is removed from the water dispersion of the inventive particles. Water may be removed, for example, by heating the water dispersion after reaction under atmospheric or reduced pressure. Specifically, a water removal method of allowing the dispersion to stand at elevated temperature, a water removal method of stirring and fluidizing the dispersion at elevated temperature, a method of spraying and dispersing the dispersion in hot air stream by means of a spray dryer, and a method utilizing a flowing heat medium. As a pretreatment before this operation, the dispersion may be concentrated by such means as heat dewatering, filtration, centrifugation, or decantation. If necessary, the dispersion may be washed with water or alcohol.

When the particles obtained by removing water from the water dispersion after reaction agglomerate together, the agglomerates are disintegrated by a crusher such as a jet mill, ball mill or hammer mill whereby silicone rubber spherical particles which are covered on their surface with the polyorganosilsesquioxane are obtained.

### [Cosmetic composition]

Another embodiment of the invention is a cosmetic composition comprising the silicone particles defined above. The silicone particles may be formulated as such into the cosmetic composition while the amount thereof may be selected as appropriate in the range of 1 to 99% by weight of the overall cosmetic composition. The silicone particles may be applied to a variety of cosmetics, preferably cosmetics applied externally to the skin such as skin care cosmetics, make-up cosmetics, antiperspirant cosmetics, and UV-protecting cosmetics and cosmetics applied externally to the hair such as hair care cosmetics. Exemplary skin care cosmetic compositions include toilet water, milky lotion, cream, cleansing agent, pack, oil liquid, massage cream, cosmetic liquid, cosmetic oil, detergent, deodorant, hand cream, lip cream, and anti-wrinkle agent. Exemplary makeup cosmetic compositions include makeup foundation, concealer, cosmetic powder, powder foundation, eye color, eye shadow, mascara, eye liner, eye blow, and lipstick. Exemplary antiperspirant cosmetic compositions include antiperspirants of roll-on, cream, solution, and stick types. Exemplary UV-protecting cosmetic compositions include sun-screen oil, sun-screen milky lotion, and sun-screen cream. Exemplary hair care cosmetic compositions include shampoo, rinse, treatment, and setting agent.

The type of the inventive cosmetic composition may be selected from among powder, oily liquid, water-in-oil emulsion, oil-in-water emulsion, non-aqueous emulsion, W/O/W and O/W/O multi-emulsions. The form of the inventive cosmetic composition may be selected from among liquid, milky lotion, cream, solid, paste, gel, powder, pressed, multilayer, mousse, spray, stick, pencil and other forms.

The inventive cosmetic composition is applicable to a variety of products including skin care, liquid foundation, powder foundation, concealer, lipstick and the foregoing products provided additionally with sunscreen performance although the products are not limited thereto as long as the cosmetic composition contains the essential component.

### [Oily component acceptable as cosmetics]

The cosmetic composition of the invention may contain various components which are commonly used in conventional cosmetics as long as the benefits of the invention are not impaired. In particular, oily components acceptable as cosmetics are preferably formulated. Oily components may be used alone or in admixture of two or more.

As the oily component, for example, hydrocarbon oils, higher alcohols, ester oils, glyceride oils, naturally occurring animal and plant oils and semi-synthetic oils, fluorinated oils, UV absorbers, non-crosslinked silicone surfactants, other surfactants, silicone oils, modified silicone oils, silicone elastomers, and oil-soluble gelling agents may be used.

### • Hydrocarbon oil

The hydrocarbon oils include straight or branched hydrocarbon oils while they may be either volatile or nonvolatile. Examples of the hydrocarbon oil include olefin oligomers (INCI), isoparaffins such as (C13, 14) isoparaffin (INCI), isododecane (INCI), undecane (INCI), dodecane (INCI), isohexadecane (INCI), hydrogenated polyisobutene (labeling name, INCI: Hydrogenated Polyisobutene), squalane (INCI), mineral oil (INCI), and alkanes such as coconut alkane (INCI) and (C13-15) alkane (INCI).

### • Higher alcohol

The higher alcohols include, for example, alcohols of 6 or more carbon atoms, preferably 10 to 30 carbon atoms. Examples of the higher alcohol include lauryl alcohol (INCI), myristyl alcohol (INCI), palmityl alcohol (INCI), stearyl alcohol (INCI), behenyl alcohol (INCI), oleyl alcohol (INCI), isostearyl alcohol (INCI), octyl dodecanol (INCI), cholesterol (INCI), phytosterol (INCI), and batyl alcohol (INCI).

### • Ester oil

Examples of the ester oil include diisobutyl adipate (labeling name, INCI: Diisobutyl Adipate), dihexyldecyl adipate (labeling name), diheptylundecyl adipate (labeling name, INCI: Diheptylundecyl Adipate), n-alkylglycol monoisostearates such as isostearyl isostearate (labeling name, INCI: Isostearyl Isostearate), isocetyl isostearate (labeling name, INCI: Isocetyl Isostearate), trimethylolpropane triisostearate (labeling name, INCI: Trimethylolpropane Triisostearate), glycol diethylhexanoate (labeling name, INCI: Glycol Diethylhexanoate), cetyl ethylhexanoate (labeling name, INCI: Cetyl Ethylhexanoate), trimethylolpropane triethylhexanoate (labeling name, INCI: Trimethylolpropane Triethylhexanoate), pentaerythrityl tetraethylhexanoate (labeling name, INCI: Pentaerythrityl Tetraethylhexanoate), cetyl octanoate (labeling name, INCI: Cetyl Ethylhexanoate), octyldodecyl esters such as octyldodecyl stearoyloxystearate (labeling name, INCI: Octyldodecyl Stearoyl Stearate), oleyl oleate (labeling name, INCI: Oleyl Oleate), octyldodecyl oleate (labeling name, INCI: Octyldodecyl Oleate), decyl oleate (labeling name, INCI: Decyl Oleate), neopentyl glycol dioctanoate (labeling name, INCI: Neopentyl Glycol Diethylhexanoate), neopentyl glycol dicaprate (labeling name, INCI: Neopentyl Glycol Dicaprate), diisostearyl malate (labeling name, INCI: Diisostearyl Malate), triethyl citrate (labeling name, INCI: Triethyl Citrate), diethylhexyl succinate (labeling name, INCI: Diethylhexyl Succinate), amyl acetate (labeling name, INCI: Amyl Acetate), ethyl acetate (labeling name, INCI: Ethyl Acetate), butyl acetate (labeling name, INCI: Butyl Acetate), isocetyl stearate (labeling name, INCI: Isocetyl Stearate), butyl stearate (labeling name, INCI: Butyl Stearate), diisopropyl sebacate (labeling name, INCI: Diisopropyl Sebacate), diethylhexyl sebacate (labeling name, INCI: Diethylhexyl Sebacate), cetyl lactate (labeling name, INCI: Cetyl Lactate), myristyl lactate (labeling name, INCI: Myristyl Lactate), isononyl isononanoate (labeling name, INCI: Isononyl Isononanoate), isotridecyl isononanoate (labeling name, INCI: Isotridecyl Isononanoate), palmitates such as isopropyl palmitate (labeling name, INCI: Isopropyl Palmitate), ethylhexyl palmitate (labeling name, INCI: Ethylhexyl Isopalmitate), and hexyldecyl palmitate (labeling name, INCI: Isocetyl Palmitate, Hexyldecyl Palmitate), cholesteryl hydroxystearate (labeling name, INCI: Cholesteryl Hydroxystearate), myristates such as isopropyl myristate (labeling name, INCI: Isopropyl Myristate), octyldodecyl myristate (labeling name, INCI: Octyldodecyl Myristate), and myristyl myristate (labeling name, INCI: Myristyl Myristate), ethylhexyl laurate (labeling name, INCI: Ethylhexyl Laurate), hexyl laurate (labeling name, INCI: Hexyl Laurate), dioctyldodecyl lauroyl glutamate (labeling name, INCI: Dioctyldodecyl Lauroyl Glutamate), and isopropyl lauroyl sarcosinate (labeling name, INCI: Isopropyl Lauroyl Sarcosinate).

Glyceride oils are also included in the ester oils, such as triethylhexanoin (INCI), glyceryl tri(caprylate/caprate) (labeling name, INCI: Caprylic/Capric Triglyceride), cocoglyceryl (INCI), triglyceryl caprylate/caprate/succinate (labeling name, INCI: Caprylic/Capric/Succinic Triglyceride), and caprylic/capric glycerides (labeling name, INCI: Caprylic/Capric Glycerides).

### • Natural animal and plant oils and semi-synthetic oils

Examples of the natural animal and plant oils and semi-synthetic oils include naturally occurring plant oils such as avocado oil (labeling name, INCI: Persea Gratissima (Avocado) Oil), linseed oil (labeling name, INCI: Linum Usitatissimum (Linseed) Seed Oil), almond oil (labeling name, INCI: Prunus Amygdalus Dulcis (Sweet Almond) Oil), perilla oil (labeling name), olive oil (labeling name, INCI: Olea Europaea (Olive) Fruit Oil), Torreya California oil (labeling name, INCI: Torreya Californica (Calfornia Nutmeg) Oil), citronella grass oil (labeling name, INCI: Cymbopogon Nardus (Citronella) Oil), kaya seed oil (labeling name, INCI: Torreya Nucifera Seed Oil), kyounin oil (labeling name, INCI: Kyounin Yu), wheat germ oil (labeling name, INCI: Triticum Vulgare (Wheat) Germ Oil), sesame oil (labeling name, INCI: Sesamum Indicum (Sesame) Seed Oil), wheat germ oil (labeling name, INCI: Triticum Vulgare (Wheat) Germ Oil); germ oils such as rice germ oil (labeling name, INCI: Oryza Sativa (Rice) Germ Oil), rice bran oil (labeling name, INCI: Oryza Sativa (Rice) Bran Oil), camellia oil (labeling name, INCI: Camellia Kissi Seed Oil), sufflower oil (labeling name, INCI: Carthamus Tinctorius (Safflower) Seed Oil), soybean oil (labeling name, INCI: Glycine Soja (Soybean) Oil), gold tea oil (labeling name, INCI: Camellia Sinensis Seed Oil), tea seed oil (labeling name, INCI: Camellia Japonica Seed Oil), evening primrose oil (labeling name, INCI: Oenothera Biennis (Evening Primrose) Oil), rapeseed oil (labeling name), corn germ oil (labeling name, INCI: Zea Mays (Corn) Germ Oil), wheat germ oil (labeling name, INCI: Triticum Vulgare (Wheat) Germ Oil); natural plant oils such as persic oil (labeling name), palm oil (labeling name, INCI: Elaeis Guineensis (Palm) Oil), palm kernel oil (labeling name, INCI: Elaeis Guineensis (Palm) Kernel Oil), castor oil (labeling name, INCI: Ricinus Communis (Castor) Seed Oil), sunflower oil (labeling name, INCI: Helianthus Annuus (Sunflower) Seed Oil), grape seed oil (labeling name, INCI: Vitis Vinifera (Grape) Seed Oil), jojoba seed oil (labeling name, INCI: Simmondsia Chinensis (Jojoba) Seed Oil), macadamia seed oil (labeling name, INCI: Macadamia Ternifolia Seed Oil), meadowfoam oil (labeling name, INCI: Limnanthes Alba (Meadowfoam) Seed Oil), cotton seed oil (labeling name, INCI: Gossypium Herbaceum (Cotton) Seed Oil), coconut oil (labeling name, INCI: Cocos Nucifera (Coconut) Oil), and peanut oil (labeling name, INCI: Archis Hypogaea (Peanut) Oil); natural animal oils such as shark liver oil (labeling name, INCI: Shark Liver Oil), cod liver oil (labeling name, INCI: Cod Liver Oil), fish liver oil (labeling name, INCI: Fish Liver Oil), turtle oil (labeling name, INCI: Turtle Oil), mink oil (labeling name, INCI: Mink Oil), and egg oil (labeling name, INCI: Egg Oil); and semi-synthetic oils and fats such as hydrogenated coconut oil (labeling name, INCI: Hydrogenated Coconut Oil) and liquid lanolin (labeling name, INCI: Lanolin Oil).

### • Fluorochemical oils

### Examples of the fluorochemical oil include perfluorodecalin (INCI), perfluorononyl dimethicone (INCI), and perfluoromethylcyclopentane (INCI).

Suitable UV absorbers include oxybenzone-1 (labeling name, INCI: Benzophenone-1), oxybenzone-2 (labeling name, INCI: Benzophenone-2), oxybenzone-3 (labeling name, INCI: Benzophenone-3), oxybenzone-4 (labeling name, INCI: Benzophenone-4), oxybenzone-5 (labeling name, INCI: Benzophenone-5), oxybenzone-6 (labeling name, INCI: Benzophenone-6), oxybenzone-9 (labeling name, INCI: Benzophenone-9), homosalate (INCI), octocrylene (INCI), t-butyl methoxydibenzoylmethane (labeling name, INCI: Butyl Methoxydibenzoylmethane), ethyl hexyl salicylate (labeling name, INCI: Ethyl Hexyl Salicylate), diethylaminohydroxybenzoyl hexyl benzoate (labeling name, INCI: Diethylamino Hydroxybenzoyl Hexyl Benzoate), polysilicone-15 (INCI), dimethoxybenzylidene dioxoimidazolidine octyl propionate (labeling name, INCI: Ethylhexyl Dimethoxybenzylidene Dioxoimidazolidine Propionate), terephthalylidene dicamphor sulfonic acid (labeling name, INCI: Terephthalylidene Dicamphor Sulfonic Acid), ethylhexyl triazone (INCI), methylbis(trimethylsiloxy)silyl isopentyl trimethoxycinnamate (labeling name, INCI: Isopentyl Trimethoxycinnamate Trisiloxane), drometrizole trisiloxane (INCI), dimethyl PABA ethylhexyl (labeling name, INCI: Ethylhexyl Dimethyl PABA), isopropyl para-methoxycinnamate (labeling name, INCI: Isopropyl Methoxycinnamate), ethylhexyl methoxycinnamate (labeling name, INCI: Ethylhexyl Methoxycinnamate), bisethylhexyloxyphenol methoxyphenyltriazine (INCI), phenylbenzimidazole sulfonic acid (labeling name, INCI: Phenylbenzimidazole Sulfonic Acid), methylene bisbenzotriazolyl tetramethylbutylphenol (INCI), glyceryl ethylhexanoate dimethoxycinnamate (labeling name, INCI: Glyceryl Ethylhexanoate Dimethoxycinnamate), glyceryl PABA (INCI), methyl diisopropylcinnamate (labeling name, INCI: Diisopropyl Methyl Cinnamate), cinoxate (INCI), ethylhexyl dimethoxybenzylidene dioxoimidazolidine propionate (labeling name, INCI: Ethylhexyl Dimethoxybenzylidene Dioxoimidazolidine Propionate), etc. Since the liquid UV-absorber is highly oil absorptive, the inventive cosmetic composition having formulated therein the liquid UV-absorber, such as ethylhexyl methoxycinnamte (labeling name, INCI: Ethylhexyl Methoxycinnamte) or ethylhexyl salicylate (labeling name, INCI: Ethylhexyl Salicylate) is highly effective for suppressing the sticky or greasy feeling or glittering inherent to the UV-absorber and improving feeling on use.

The non-crosslinked silicone surfactants are straight or branched silicones in which some methyl groups on the backbone are substituted with hydrophilic groups such as polyethylene glycol and polyglycerin, for example, straight or branched polyoxyethylene-modified organopolysiloxanes, straight or branched polyoxyethylene/polyoxypropylene-modified organopolysiloxanes, straight or branched polyoxyethylene/alkyl-co-modified organopolysiloxanes, straight or branched polyoxyethylene/polyoxypropylene/alkyl-co-modified organopolysiloxanes, straight or branched polyglycerin-modified organopolysiloxanes, straight or branched polyglycerin/alkyl-co-modified organopolysiloxanes, and straight or branched pyrrolidone-modified organopolysiloxanes. In these surfactants, the amount of hydrophilic polyoxyethylene, polyoxyethylene polyoxypropylene or polyglycerin residues incorporated preferably accounts for 10 to 70% by weight of the molecule. Included are PEG-11 methyl ether dimethicone (INCI), PEG/PPG-20/22 butyl ether dimethicone (INCI), PEG-3 dimethicone (INCI), PEG-10 dimethicone (INCI), PEG-9 polydimethylsiloxyethyl dimethicone (INCI), lauryl PEG-9 polydimethylsiloxyethyl dimethicone (INCI), cetyl PEG/PPG-10/1 dimethicone (INCI), polyglyceryl-3 polydimethylsiloxyethyl dimethicone (INCI), and lauryl polyglyceryl-3 polydimethylsiloxyethyl dimethicone (INCI).

Commercially available examples include KF-6011, KF-6011P, KF-6012, KF-6015, KF-6017, KF-6043, KF-6028, KF-6038, KF-6048, KF-6100, KF-6104, KF-6106, KF-6105, and KF-6115 by Shin-Etsu Chemical Co., Ltd. The amount of the non-crosslinked silicone surfactants formulated.

### • Other surfactants

The other surfactant may be nonionic, anionic, cationic or ampholytic. The surfactant is not particularly limited as long as it is commonly blended in cosmetics. When crosslinked organopolysiloxanes such as partially crosslinked polyether-modified silicones or partially crosslinked polyglycerine-modified silicones are used, suitable compositions comprising the crosslinked organopolysiloxane and an oil which is liquid at room temperature include gel mixtures containing a gelling component such as the crosslinked organopolysiloxane and an oil component such as cyclopentasiloxane (INCI), dimethicone (INCI), mineral oil (INCI), isododecane (INCI), triethylhexanoin (INCI) or squalane (INCI). Included are (dimethicone)/(PEG-10/15) crosspolymer (INCI), (PEG-15/lauryl dimethicone) crosspolymer (INCI), (PEG-10/lauryl dimethicone) crosspolymer (INCI), (PEG-15/lauryl polydimethylsiloxyethyl dimethicone) crosspolymer (INCI), (dimethicone/polyglycerine-3) crosspolymer (INCI), (lauryldimethicone/polyglycerine-3) crosspolymer (INCI), and (polyglycerine-3/lauryl polydimethylsiloxyethyl dimethicone) crosspolymer (INCI).

Commercially available examples of the crosslinked organopolysiloxane which is swollen with the liquid oil include KSG-210, KSG-240, KSG-270, KSG-310, KSG-320, KSG-330, KSG-340, KSG-320Z, KSG-350Z, KSG-710, KSG-810, KSG-820, KSG-830, KSG-840, KSG-820Z, and KSG-850Z from Shin-Etsu Chemical Co., Ltd.

The silicone oils encompass cyclic and straight dimethylpolysiloxanes having a kinematic viscosity at 25°C of at least 4 mm²/s and cyclic and straight dimethylpolysiloxanes having a kinematic viscosity at 25°C of at least 4 mm²/s, specifically cyclopentasiloxane (INCI), cyclohexasiloxane (INCI), and dimethicone (INCI).

Commercially available examples of the silicone oil include KF-995 and KF-96A-6cs from Shin-Etsu Chemical Co., Ltd.

The modified silicone oil refers to straight dimethylpolysiloxane and modified silicone oils having a kinematic viscosity at 25°C of less than 4 mm²/s. Examples include alkyl-modified silicones having a kinematic viscosity at 25°C of 1.5 to 2 mm²/s, such as dimethicone (INCI), trisiloxane (INCI), methyltrimethicone (INCI), ethyltrisiloxane (INCI) and hexyldimethicone (INCI); long chain alkyl-modified silicones such as caprylyl methicone (INCI); aromatic group-modified silicones, typically straight or branched organopolysiloxanes such as phenyltrimethicone (INCI), diphenyldimethicone (INCI), diphenylsiloxyphenyltrimethicone (INCI), and tetraphenyldimethyldisiloxane (INCI) and cyclic organopolysiloxanes such as tetramethyltetraphenylcyclotetrasiloxane; amino-modified organopolysiloxanes such as amodimethicone (INCI) and aminopropyldimethicone (INCI); pyrrolidone-modified organopolysiloxanes such as PCA dimethicone (INCI); pyrrolidonecarboxylic acid-modified organopolysiloxanes, amino acid-modified silicones, and fluorine-modified silicones.

Commercially available examples of the modified silicone oil include KF-56A, KF-4422, and KF-96L-2cs from Shin-Etsu Chemical Co., Ltd.

Included in the silicone elastomer are gel mixtures containing a gelling component, for example, unmodified or modified partially crosslinked organopolysiloxanes such as unmodified partially crosslinked organopolysiloxanes, alkyl-modified partially crosslinked organopolysiloxanes, or silicone-branched alkyl-modified partially crosslinked organopolysiloxanes, and an oil component such as cyclopentasiloxane (INCI), dimethicone (INCI), mineral oil (INCI), isododecane (INCI), triethylhexanoin (INCI), or squalane (INCI). Exemplary are (dimethicone/vinyl dimethicone) crosspolymer (INCI), (dimethicone/phenylvinyl dimethicone) crosspolymer (INCI),
(vinyldimethicone/lauryldimethicone) crosspolymer (INCI), and
(laurylpolydimethylsiloxyethyl dimethicone/bis-vinyl dimethicone) crosspolymer (INCI).

Commercially available examples of the silicone elastomer include KSG-15, KSG-1510, KSG-16, KSG-1610, KSG-19, KSG-016F, KSG-18A, KSG-41A, KSG-42A, KSG-43, KSG-44, KSG-042Z, KSG-045Z, and KSG-048Z from Shin-Etsu Chemical Co., Ltd.

Examples of the silicone resin include silicone rubbers such as gum-like dimethylsiloxane/methylphenylsiloxane copolymers; solutions of silicone gum or rubber in dimethicone (INCI), cyclopentasiloxane (INCI) or isododecane (INCI); and trimethylsiloxysilicic acid (labeling name, INCI: Trimethylsiloxysilicate). Also included are solutions of acrylic silicone resins in the form of acryloyl/silicone graft copolymers or acryloyl/silicone block copolymers in butyl acetate (INCI), dimethicone (INCI), cyclopentasiloxane (INCI) or isododecane (INCI); (acrylate/dimethicone) copolymer (INCI), isododecane (INCI) solution of silicone-modified polynorbornene, (norbornene/tris(trimethylsiloxy)silylnorbornene) copolymer (INCI), cyclopentasiloxane (INCI) or isododecane (INCI) solution of silicone-modified pullulan, and tri(trimethylsiloxy)silylpropylcarbamate pullulan (labeling name, INCI: Trimethylsiloxysilylcarbamoyl Pullulan).

Commercially available examples of the silicone resin include KF-7312J, KP-545, NBN-30-ID, and TSPL-30-ID from Shin-Etsu Chemical Co., Ltd.

Suitable oil-soluble gelling agents include metal soaps such as aluminum stearate, magnesium stearate, and zinc myristate, amino acid derivatives such as lauroyl glutamic acid (labeling name, INCI: Lauroyl Glutamic Acid) and α,y-di-n-butylamine, dextrin fatty acid esters such as dextrin palmitate (INCI), dextrin isostearate (INCI), dextrin myristate (INCI), inulin stearate (INCI), and dextrin 2-ethylhexanoate palmitate, sucrose fatty acid esters such as sucrose palmitate and sucrose stearate, fructooligosaccharide fatty acid esters such as fructooligosaccharide stearate and fructooligosaccharide 2-ethylhexanoate, benzylidene derivatives of sorbitol such as monobenzylidene sorbitol and dibenzylidene sorbitol, organo-modified clay minerals of hectorite such as disteardimonium hectorite (INCI) and stearalkonium hectorite (INCI); stearalkonium bentonite (INCI), esters which are pasty at 25°C, vaseline (INCI), lanolin (INCI), and solid waxes. Suitable silicone waxes include (acrylate/stearyl acrylate/dimethicone methacrylate) copolymer (labeling name, INCI: Acrylates/Stearyl Acrylate/ Dimethicone Methacrylate Copolymer).

Exemplary of the commercially available silicone wax is KP-561P from Shin-Etsu Chemical Co., Ltd.

When blended in the inventive cosmetic composition, the amount of the oily component blended is preferably 1 to 98% by weight of the overall cosmetic composition.

### [Oily gel composition]

Although the inventive cosmetic composition may contain the silicone spherical particles as a cosmetic component without further treatment as mentioned above, it is preferred, for the purpose of acquiring a feeling on use desired for cosmetics, to separately prepare an oily gel composition containing the silicone spherical particles and the oily component mentioned above, and to prepare a cosmetic composition containing the silicone spherical particles and the oily component in the form of the oily gel composition. In general, various oily components are used in suitable combination in cosmetics. In the step of preparing a cosmetic composition, if the silicone spherical particles are added alone to a mixture of oily components, swelling of the silicone microparticles develops in the mixture. In contrast, even when the blending composition of the oily component is eventually the same, the swelling of silicone spherical particles is previously controlled using part of the oily components whereby the cosmetic composition having the desired feeling on use is obtained. That is, in one exemplary procedure, an oily gel composition containing some oily components capable of inhibiting swelling of silicone microparticles, selected from the oily components to be formulated in the cosmetic composition, and the silicone spherical particles is previously formed, whereby swelling of silicone spherical particles is inhibited. Using such an oily gel composition, a cosmetic composition having a powdery feeling on use is obtainable. Inversely, when an oily gel composition containing the oily component having sufficient swell and the silicone spherical particles is formed, then swelling of silicone spherical particles is enlarged. Using such an oily gel composition, there are obtained a cosmetic composition having a silky feeling on use and a cosmetic composition developing a soft, elastic film feeling.

In the oily gel composition, the oily component is structured by the silicone spherical particles. The phrase that "the oily component in structured state" means that the oily component is hardened, gelled, pasted or simply thickened by the silicone spherical particles. That is, the oily component structured by the silicone spherical particles does not flow away from the silicone spherical particles under the impetus of its own weight. The oily component gelled or pasted by the silicone spherical particles has an increased viscosity. Since the hardened, gelled, pasted or simply thickened state mentioned above refers to the state at the temperature at which the oily component is liquid, the oily component may be liquid, semi-solid or solid at normal temperature. That is, even when the oily component is semi-solid or solid at normal temperature, the oily component can be hardened, gelled, pasted or simply thickened by the silicone microparticles as long as the oily component can be liquefied by heating at any temperature.

The method of preparing the oily gel composition is not particularly limited. As long as the oily component is liquid at normal temperature, the oily gel composition can be obtained by agitating and mixing the silicone microparticles and the oily component. By the agitating and mixing step, the silicone spherical particles are dispersed in the oily component while the silicone spherical particles absorb the oily component and reach a less flowing state, i.e., "the oily component in structured state." Also, when the oily component which is solid or semi-solid at normal temperature is used, the oily gel composition can be obtained by agitating and mixing the silicone spherical particles and the oily component at the temperature at which the oily component can be liquefied. Agitation may be performed without a need for high shear force, namely by a force sufficient to uniformly disperse the silicone spherical particles in the oily component. Examples of the agitating machine include propeller impellers, plate impellers, anchor mixers, planetary mixers, and kneading extruders.

The ratio of the silicone spherical particles to the oily component in the oily gel composition is preferably from 5/95 to 90/10 in weight ratio. The oily gel composition may be used alone or in admixture of two or more. In the inventive cosmetic composition, the amount of the oily gel composition blended is preferably in the range of 1.1 to 98.1% by weight of the overall cosmetic composition.

### [Carrier acceptable as cosmetics]

In a preferred embodiment of the invention, the cosmetic composition further contains a carrier acceptable as cosmetics in such a state that the oily gel composition is mixed with the carrier. The carrier may be used alone or in admixture of two or more. As used herein, the "carrier" refers to an oily component which is acceptable as cosmetics and can be mixed with the oily gel composition and a component other than the oily component which is acceptable as cosmetics and can be mixed with the oily gel composition. Examples of the component other than the oily component which is acceptable as cosmetics and can be mixed with the oily gel composition include (a) water, (b) a humectant, (c) a water-soluble polymer, (d) a powder, and other additives, which may be used alone or in admixture of two or more.

### (a) Water

Examples of water as component (a) include purified water, distillates derived from fruits and plants, sea water (labeling name, INCI: Sea Water), hot spring water (labeling name), and peat water (labeling name, INCI: Peat Water), which are commonly used in cosmetics. The content of water blended which depends on the form of the cosmetic composition is preferably 1 to 95% by weight of the overall cosmetic composition.

### (b) Humectant

Suitable humectants as component (b) include sugar alcohols such as sorbitol (INCI), maltose (INCI), and xylitol (INCI); polyhydric alcohols such as BG (labeling name, INCI: Butylene Glycol), PG (labeling name, INCI: Propylene Glycol), pentylene glycol (INCI), 1,10-decanediol (INCI), octanediol (INCI), 1,2-hexanediol (INCI), erythritol (INCI), glycerin (INCI name), diglycerin (INCI), and polyethylene glycol; compounds having an alcoholic hydroxy group such as glucose (INCI), glyceryl glucoside (INCI), betaine (INCI), sodium chondroitin sulfate (labeling name, INCI: Sodium Chondroitin Sulfate), PCA-Na (labeling name, INCI: Sodium PCA), methylgluces-10 (INCI), and methyl gluces-20 (INCI). When blended, the content of component (b) is preferably selected in the range of 0.1 to 98% by weight of the overall cosmetic composition.

### (c) Water-soluble polymer

Suitable water-soluble polymers as component (c) include naturally occurring water-soluble polymers such as carrageenan (INCI), sodium hyaluronate (labeling name, INCI: Sodium Hyaluronate), xanthan gum (INCI), and agar (labeling name, INCI: Agar); semi-synthetic water-soluble polymers such as hydroxyethyl cellulose (INCI), hydroxypropyl methylcellulose (INCI), and carboxymethyl cellulose (INCI); synthetic water-soluble polymers such as polyvinyl alcohol (INCI), carbomer (INCI), acrylate/(C10-30)alkyl acrylate crosspolymer (INCI), ammonium acryloyldimethyltaurate/VP copolymer (INCI), sodium acrylate/sodium acryloyldimethyltaurate copolymer (INCI), and hydroxyethyl acrylate/sodium acryloyldimethyltaurate copolymer (INCI); and inorganic water-soluble polymers such as bentonite (INCI) and laponite (INCI). When blended, the content of component (c) is preferably in the range of 0.1 to 25% by weight of the overall cosmetic composition.

### (d) Powder

Suitable powders as component (d) include microparticulate metal oxide powders, hydrophobized coloring pigments, organic colorants, inorganic powders, metal powders, metal soaps, organic powders, inorganic/organic composite powders, and inorganic/inorganic composite powders.

The microparticulate metal oxide used herein is one or more selected from microparticulate titanium oxide (labeling name, INCI: Titanium Dioxide), iron-containing titanium oxide, zinc oxide (labeling name, INCI: Zinc Oxide), cerium oxide (labeling name, INCI: Cerium Oxide), and composites thereof, which may be used alone or in admixture. The metal oxide may also be a composite powder with another powder. The powder has an average primary particle size of preferably up to 200 nm, more preferably up to 120 nm. If the particle size is larger than the limit, the UV-protecting function is degraded, with white spots being left. It is noted that the average primary particle size can be determined from a photo taken under a transmission electron microscope.

The microparticulate metal oxide may be used in untreated state or after a well-known surface treatment commonly used in cosmetics. For example, suitable inorganic treatments include coating with silica (INCI), coating with alumina (INCI), and coating with Al hydroxide (labeling name, INCI: Aluminum Hydroxide). Suitable organic treatments include coating with silanes or silylating agents such as triethoxycaprylylsilane (INCI), coating with silicone oils such as dimethicone (INCI), methicone (INCI), hydrogen dimethicone (INCI), triethoxysilylethyl polydimethylsiloxyethyl hexyl dimethicone (INCI), and acrylate/tridecyl acrylate/triethoxysilylpropyl methacrylate/dimethicone methacrylate copolymer (labeling name, INCI: Acrylates/Tridecyl Acrylate/Triethoxysilylpropyl Methacrylate/Dimethicone Methacrylate Copolymer), waxes, paraffins, organic fluorine compounds such as perfluoroalkyl phosphoric acid salts, surfactants, amino acids such as N-acylglutamic acid, and metal soaps such as Al stearate (labeling name, INCI: Aluminum Stearate) and Mg myristate (labeling name, INCI: Magnesium Myristate). As the silicone treating agent, the silicone powder treating agents described in JP 3912961 are preferably used. In particular, triethoxysilylethyl polydimethylsiloxyethyl hexyl dimethicone (INCI) which is a dimethylpolysiloxane having triethoxysilyl, polydimethylsiloxyethyl and hexyl groups on side chains, or the like is advantageously used because it exerts a high affinity even when the dispersing medium in which the highly hydrophobized coloring pigment is dispersed is a mixture of silicone and hydrocarbon. It is noted that the surface hydrophobizing agent may be used alone or in admixture of two or more.

Commercially available examples of the silicone powder treating agent include KF-96AK series, KF-99P, KF-9901, KF-9908, KF-9909, and KP-574 from Shin-Etsu Chemical Co., Ltd. Commercially available examples of the coloring pigment subjected to hydrophobic surface treatment with the silicone powder treating agent include KTP-09W, KTP-09R, KTP-09Y, and KTP-09B from Shin-Etsu Chemical Co., Ltd.

As the surface-treated microparticulate metal oxide, commercially available products may be used. For example, microparticulate titanium oxide is commercially available under the trade name of STR-100C-LP, STR-100A-LP, STR-100W, STR-100W-LP, STR-100C-LF, STR-40-LP (Sakai Chemical Industry Co., Ltd.), MT-01, MT-05, MT-100Z, MT-100TV, MT-100AQ, MT-150EX, MT-500B, MT-505SAS, MT-700B, MT-014Z, SMT-500SAS (Tayca Corp.), and ST-455, ST-455WS, ST-457ECS and ST-495M (Titan Kogyo Ltd.). Microparticulate zinc oxide is commercially available under the trade name of FINEX-50S-LP2, FINEX-30S-LP2, FINEX-50W, FINEX-30W, FINEX-50W-LP2, FINEX-52W-LP2, FINEX-30W-LP2, FINEX-33W-LP2, FINEX-50-LPT, FINEX-25-LPT, FINEX-50S-LPT, FINEX-30S-LPT (Sakai Chemical Industry Co., Ltd.), MZ-150, MZ-200, MZ-300, MZ-306X, MZ-500HP, MZ-505T, MZ-506X, MZY-203S, MZY-210M3S, TMZ-HA1, and MZX-5080TS (Tayca Corp.).

The coloring pigment for the hydrophobized coloring pigment is not particularly limited as long as it is commonly used in cosmetics for the coloring purpose. Examples include red iron oxide (labeling name, INCI: Iron Oxides), yellow iron oxide (labeling name, INCI: Iron Oxides), white titanium oxide of pigment grade (labeling name, INCI: Titanium Dioxide), black iron oxide (labeling name, INCI: Iron Oxides), Ultramarine (labeling name, INCI: Ultramarines), Prussian blue (labeling name, INCI: Ferric Ferrocyanide, Ferric Ammonium Ferrocyanide), manganese violet (labeling name, INCI: Manganese Violet), cobalt titanate (labeling name, INCI: Cobalt Titanium Oxide), chromium hydroxide (labeling name, INCI: Chromium Hydroxide Green), chromium oxide (labeling name, INCI: Chromium Oxide Greens), Al/cobalt oxide (labeling name, INCI: Cobalt Aluminum Oxide), cobalt titanate (labeling name, INCI: Cobalt Titanium Oxide), fired titanium/titanium oxide (labeling name, INCI: Titanium/Titanium Dioxide), Li/cobalt titanate (labeling name, INCI: Lithium Cobalt Titanate), cobalt titanate (labeling name, INCI: Cobalt Titanium Oxide), sintered iron oxide/titanium oxide (labeling name), composites doped with a different metal such as iron oxide-doped titanium oxide (labeling name, INCI: Iron Oxides, Titanium Dioxide), titanium nitride (labeling name, INCI: Titanium Nitride), ferrous hydroxide (labeling name, INCI: Iron Hydroxide), inorganic brown pigments such as γ-iron oxide, inorganic yellow pigments such as loess, colored pigments such as lake form of tar dyes and lake form of natural dyes. Any of the foregoing may be used.

Examples of the organic colorant include tar dyes, specifically Red #3, Red #104(1) (labeling name, INCI: Red 28, Red 28 Lake), Red #106, Red #201 (labeling name, INCI: Red 6), Red #202 (labeling name, INCI: Red 7), Red #204, Red #205, Red #220 (labeling name, INCI: Red 34), Red #226 (labeling name, INCI: Red 30), Red #227 (labeling name, INCI: Red 33, Red 33 Lake), Red #228 (labeling name, INCI: Red 36), Red #230(1) (labeling name, INCI: Red 22, Red 22 Lake), Red #230(2) (labeling name), Red #401 (labeling name), Red #505 (labeling name), Yellow #4 (labeling name, INCI: Yellow 5), Yellow #5 (labeling name, INCI: Yellow 6, Yellow 6 Lake), Yellow #202(1) (labeling name, INCI: Yellow 8), Yellow #203 (labeling name, INCI: Yellow 10, Yellow 10 Lake), Yellow #204 (labeling name, INCI: Yellow 11), Yellow #401 (labeling name), Blue #1 (labeling name, INCI: Blue 1, Blue 1 Lake), Blue #2, Blue #201, Blue #205 (labeling name, INCI: Blue 4), Blue #404 (labeling name), Blue #404 (labeling name), Green #3 (labeling name, INCI: Green 3, Green 3 Lake), Green #201 (labeling name, INCI: Green 5), Green #202 (labeling name, INCI: Green 6), Green #204 (labeling name, INCI: Green 8), Green #205 (labeling name), Orange #201 ((labeling name, INCI: Orange 5), Orange #203 (labeling name, INCI: Pigment Orange 5), Orange #204 (labeling name), Orange #205 (labeling name, INCI: Orange 4, Orange 4 Lake), Orange #206 (labeling name, INCI: Orange 10), Orange #207 (labeling name, INCI: Orange 11); and natural dyes, specifically cochineal (INCI), laccaic acid (labeling name, INCI: Laccaic Acid), safflower red (labeling name, INCI: Garthamus Tinctorius (Safflower) Flower Extract), red root extract (labeling name, INCI: Lithospermum Officinale Root Extract), gardenia yellow (labeling name), and gardenia blue (labeling name, INCI: Hydrolyzed Gardenia Florida Extract).

Examples of the inorganic powder include microparticles of zirconium oxide (labeling name, INCI: Zirconium Dioxide), zinc oxide (labeling name, INCI: Zinc Oxide), cerium oxide (labeling name, INCI: Cerium Oxide), magnesium oxide (labeling name, INCI: Magnesium Oxide), barium sulfate (labeling name, INCI: Barium Sulfate), calcium sulfate (labeling name, INCI: Calcium Sulfate), magnesium sulfate (labeling name, INCI: Magnesium Sulfate), calcium carbonate (labeling name, INCI: Calcium Carbonate), magnesium carbonate (labeling name, INCI: Magnesium Carbonate), talc (INCI), mica (INCI), kaolin (INCI), synthetic fluorphlogopite (labeling name, INCI: Synthetic Fluorphlogopite), synthetic gold mica iron (labeling name), black mica (labeling name, INCI: Biotite), potassium silicate (labeling name, INCI: Potassium Silicate), silica (INCI), aluminum silicate (labeling name, INCI: Aluminum Silicate), magnesium silicate (labeling name, INCI: Magnesium Silicate), Al/Mg silicate (labeling name, INCI: Magnesium Aluminum Silicate), calcium silicate (labeling name, INCI: Calcium Silicate), Al/Ca/Na silicate (labeling name, INCI: Aluminum Calcium Sodium silicate), Li/Mg/Na silicate (labeling name, INCI: Lithium Magnesium Sodium Silicate), Na/Mg silicate (labeling name, INCI: Sodium Magnesium Silicate), Ca/Al borosilicate (labeling name, INCI: Calcium Aluminum Borosilicate), Ca/Na borosilicate (labeling name, INCI: Calcium Sodium Borosilicate), hydroxyapatite (INCI), bentonite (INCI), montmorillonite (INCI), hectorite (INCI), zeolite (INCI), alumina (INCI), Al hydroxide (labeling name, INCI: Aluminum Hydroxide), boron nitride (labeling name, INCI: Boron Nitride), and glass (labeling name, INCI: Glass). Also, examples of the inorganic coloring pearly pigment include pearlescent agents such as mica (INCI) coated with titanium oxide (labeling name, INCI: Titanium Dioxide) and synthetic fluorphlogopite (labeling name, INCI: Synthetic Fluorphlogopite) coated with titanium oxide (labeling name, INCI: Titanium Dioxide), pearlescent pigments such as bismuth oxychloride (labeling name, INCI: Bismuth Oxychloride) coated with titanium oxide (labeling name, INCI: Titanium Dioxide), talc (labeling name, INCI: Talc) coated with titanium oxide (labeling name, INCI: Titanium Dioxide), fish scale flakes, coloring mica coated with titanium oxide (labeling name, INCI: Titanium Dioxide), which may either be untreated or have undergone well-known surface treatment commonly used for cosmetics.

Examples of the metal powder include metal microparticles such as aluminum (labeling name, INCI: Aluminum, Aluminum Powder), copper (labeling name, INCI: Copper Powder), and silver (labeling name, INCI: Silver Powder), and gold (labeling name, INCI: Gold).

Examples of the metal soap include zinc stearate (labeling name, INCI: Zinc Stearate), Al stearate (labeling name, INCI: Aluminum Stearate), Ca stearate (labeling name, INCI: Calcium Stearate), Mg stearate (labeling name, INCI: Magnesium Stearate), zinc myristate (labeling name, INCI: Zinc Myristate), Mg myristate (labeling name, INCI: Magnesium Myristate), Zn/Na cetylphosphate (labeling name, INCI: Sodium Zinc Cetyl Phosphate), K cetylphosphate (labeling name, INCI: Potassium Cetyl Phosphate) in powder form.

Examples of the organic powder include silicone, polyamide, polyacrylic acid-acrylate, polyester, polyethylene (INCI), polypropylene (INCI), polystyrene (INCI), styrene-acrylic acid copolymers, divinylbenzene-styrene copolymers, polyurethane, vinyl resins, urea resins, melamine resins, benzoguanamine, polymethyl benzoguanamine, tetrafluoroethylene, polymethyl methacrylate, cellulose (INCI), silk (INCI), nylon (labeling name), phenolic resins, epoxy resins, polycarbonate, etc. in powder form. In particular, as the silicone, silicone resin particles, polymethylsilsesquioxane (INCI), silicone rubber powder, silicone resin-coated silicone rubber powder, (vinyldimethicone/methicone silsesquioxane) crosspolymer (INCI), diphenyl dimethicone/vinyldiphenyl dimethicone/silsesquioxane) crosspolymer (INCI), polysilicone-1 crosspolymer (INCI), and polysilicone-22 (INCI).

Examples of the commercially available silicone powder include KMP-590, KMP-591, KMP-592, KMP-597, KMP-598, KSP-100, KSP-101, KSP-102, KSP-105, KSP-300, KSP-411, KSP-441, KM-9729, and KM-440 from Shin-Etsu Chemical Co., Ltd.

Useful powders other than the powder of silicone as organic powder include powders which are surface-treated with silanes or silylating agent such as triethoxycaprylylsilane (INCI), silicone oils such as dimethicone (INCI), methicone (INCI), hydrogen dimethicone (INCI), triethoxysilylethyl polydimethylsiloxyethyl dimethicone (INCI), triethoxysilylethyl polydimethylsiloxyethyl hexyl dimethicone (INCI), and acrylate/tridecyl acrylate/triethoxysilylpropyl methacrylate/dimethicone methacrylate copolymer (labeling name, INCI: Acrylates/Tridecyl Acrylate/Triethoxysilylpropyl Methacrylate/Dimethicone Methacrylate Copolymer), waxes, paraffins, organic fluorine compounds such as perfluoroalkyl phosphoric acid salts, surfactants, amino acids such as N-acylglutamic acid, and metal soaps such as aluminum stearate and magnesium myristate.

Other additives include antiperspirants, preservatives, antibacterial agents, fragrances, salts, antioxidants, pH modifiers, chelating agents, refresheners, anti-inflammatory agents, skin-improving agents (brightening or whitening agents, cell activators, anti-skin-roughening agents, blood flow promoting agents, skin astringents, and anti-seborrheic agents), vitamins, amino acids, nucleic acids, hormones, inclusion compounds, hair-setting agents, and the like.

Examples of the antiperspirant include aluminum halohydrates such as aluminum chlorohydrate and aluminum chlorohydrate allantoinate, aluminum halides such as aluminum chloride, aluminum allantoinates, tannic acid, persimmon, aluminum potassium sulfate, zinc oxide, zinc p-phenolsulfonate, aluminum potassium sulfate, aluminum zirconium tetrachlorohydrate, and aluminum zirconium trichlorohydrex glycine. The preferred components which exert a higher effect include aluminum halohydrates, aluminum halides, and complexes or mixtures thereof with zirconium oxyhalides and zirconium hydroxyhalides (e.g., aluminum zirconium tetrachlorohydrate and aluminum zirconium trichlorohydrex glycine.

Suitable preservatives and antibacterial agents include alkyl p-hydroxybenzoates, benzoic acid, sodium benzoate, sorbic acid, potassium sorbate, salicylic acid, isopropyl methyl phenol, phenol, p-chloro-m-cresol, hexachlorophene, benzalkonium chloride, chlorohexidine chloride, trichlorocarbanilide, iodopropynyl butylcarbamate, polylysine, photosensitizers, silver, and plant extracts.

Fragrances include natural fragrances and synthetic fragrances. Suitable natural fragrances include plant fragrances separated from flowers, leaves, stems, and pericarps, and animal fragrances such as musk and civet. Suitable synthetic fragrances include hydrocarbons such as monoterpene; alcohols such as aliphatic alcohols and aromatic alcohols; aldehydes such as terpene aldehydes and aromatic aldehydes; ketones such as alicyclic ketones; esters such as terpene esters; lactones; phenols; oxides; nitrogen-containing compounds; and acetals.

Examples of the antioxidant include, but are not particularly limited to, carotenoid, ascorbic acid and salts thereof, ascorbyl stearate, tocophenol, tocopherol acetate, tocopherol, p-t-butylphenol, butyl hydroxyanisole, dibutyl hydroxytoluene, phytic acid, ferulic acid, thiotaurine, hypotaurine, sulfites, erythorbic acid and salts thereof, chlorogenic acid, epicatechin, epigallocatechin, epigallocatechin gallate, apigenin, kaempferol, myricetin, and quercetin.

Suitable acidity regulators include Na hydroxide (labeling name, INCI: Sodium hydroxide), K hydroxide (labeling name, INCI: Potassium hydroxide), arginine hydroxide (labeling name, INCI: Arginine), citric acid (labeling name, INCI: Citric Acid), Na citrate (labeling name, INCI: Sodium Citrate), and lactic acid citric acid (labeling name, INCI: Lactic Acid).

Suitable chelating agents include alanine, sodium EDTA, sodium polyphosphate, sodium metaphosphate, and phosphoric acid.

Suitable refreshing agents include L-menthol, camphor and menthyl lactate.

Suitable vitamins include vitamin A family such as vitamin A oil, retinol, retinol acetate, retinol palmitate; vitamin B family, specifically vitamin B2 family such as riboflavin, riboflavin butyrate, and flavin adenine nucleotide, vitamin B6 family such as pyridoxine hydrochloride, pyridoxine dioctanoate, pyridoxine tripalmitate, vitamin B12 and derivatives thereof, vitamin B15 and derivatives thereof; vitamin C family such as L-ascorbic acid, L-ascorbic acid dipalmitic acid ester, L-ascorbic acid-2-sodium sulfate, and dipotassium L-ascorbic acid phosphoric acid diester; vitamin D family such as ergocalciferol and cholecalciferol; vitamin E family such as α-tocopherol, β-tocopherol, γ-tocopherol, dl-α-tocopherol acetate, dl-α-tocopherol nicotinate, and dl-α-tocopherol succinate; nicotinic acids such as nicotinic acid, benzyl nicotinate, and nicotinic amide; vitamin H, vitamin P, pantothenic acids such as calcium pantothenate, D-pantothenyl alcohol, pantothenyl ethyl ether, acetylpantothenyl ethyl ether; and biotin.

Suitable amino acids include glycine, valine, leucine, isoleucine, serine, threonine, phenylalanine, arginine, lysine, aspartic acid, glutamic acid, cystine, cysteine, methionine, and tryptophan. Typical of the nucleic acid is deoxyribonucleic acid. Suitable hormones include estradiol and ethenyl estradiol. Typical of the clathrate compound is cyclodextrin.

Suitable hair setting agents include ampholytic, anionic, cationic and nonionic polymers. Useful examples include ampholytic acrylic polymers, for example, polyvinylpyrrolidone polymers such as polyvinylpyrrolidone and vinylpyrrolidone/vinyl acetate copolymers, acidic vinyl ether polymers such as methyl vinyl ether/maleic anhydride alkyl half-ester copolymers, acidic polyvinyl acetate polymers such as vinyl acetate/crotonic acid copolymers, (meth)acrylic acid/alkyl (meth)acrylate copolymers, acidic acrylic polymers such as (meth)acrylic acid/alkyl (meth)acrylate/alkyl acrylamide copolymers, N-methacryloylethyl-N,N-dimethylammonium/α-N-methylcarboxybetaine/alkyl (meth)acrylate copolymers, and hydroxypropyl (meth)acrylate/butylaminoethyl methacrylate/octylamide acrylate copolymers. Naturally occurring high-molecular-weight compounds such as cellulose or derivatives thereof, keratin or derivatives thereof, collagen or derivatives thereof are also useful.

### EXAMPLES

Examples are shown below for illustrating the invention, but the invention is not limited thereto. Hereinafter, the viscosity is a kinematic viscosity measured at 25°C by a capillary viscometer. Percent (%) indicative of concentration and content is by weight. The amount of product name is the amount of the product blended. The volume average particle size is measured by a particle size distribution analyzer (Multisizer 3, Beckman Coulter) according to the electric resistance method.

### [Examples 1 to 3 and Comparative Examples 1 and 2: Silicone particles]

### [Example 1]

A glass beaker of 1 L volume was charged with 192 g of methylphenylvinylpolysiloxane (A1) having a kinematic viscosity of 560 mm²/s (and having a phenyl group content of 12.5 mol% based on silicon-bonded monovalent hydrocarbon groups exclusive of vinyl groups), represented by the average formula (13) shown below, and 8 g (to provide 1.14 SiH groups per vinyl group) of methylhydrogenpolysiloxane (B1) having a kinematic viscosity of 9 mm²/s (and having a phenyl group content of 14.3 mol% based on silicon-bonded monovalent hydrocarbon groups), represented by the average formula (14) shown below, which were stirred and dissolved using a homo-mixer at 2,000 rpm. Then, 1.2 g of polyoxyethylene lauryl ether (molar amount of ethylene oxide added = 9 mol) and 41 g of water were added to the solution, which was stirred using the homo-mixer at 6,000 rpm, with conversion to oil-in-water type and a viscosity buildup being acknowledged. Stirring was continued for a further 15 minutes. With stirring at 2,000 rpm, 755 g of water was added, obtaining a uniform white emulsion. The emulsion was transferred to a glass flask of 1 L volume equipped with an agitator having an anchor shape impeller and conditioned at 15-20°C. With stirring, a mixed/dissolved mixture of 0.7 g of an isododecane solution of platinum/vinyl-containing disiloxane complex (platinum content 0.5%) and 1.0 g of polyoxyethylene lauryl ether (molar amount of ethylene oxide added = 9 mol), and 1.4 g of polyoxyethylene lauryl ether (molar amount of ethylene oxide added = 4 mol) were added to the emulsion, which was stirred at the temperature for 1 hour. The mixture was heated at 30-40°C, after which stirring was performed at the temperature for 5 hours, obtaining a water dispersion of silicone rubber particles.

The silicone rubber particles were observed under an optical microscope, finding a spherical shape and a volume average particle size of 5 µm.

547 g of the water dispersion of silicone rubber spherical particles was transferred to a glass flask of 2 L volume equipped with an agitator having an anchor shape impeller. Then, 407 g of water, 20 g of 28% ammonia water, and 1 g of 40% polydimethylmethylene piperidinium chloride aqueous solution (trade name: ME Polymer H40W, Toho Chemical Industry Co., Ltd.) were added. At this point of time, the solution was at pH 11.2. After the solution was conditioned at 5-10°C, 25 g of methyltrimethoxysilane (corresponding to 11.3 parts by weight of polymethylsilsesquioxane after hydrolytic condensation reaction per 100 parts by weight of silicone rubber spherical particles) was added dropwise over 20 minutes. With the liquid kept at a temperature of 5-10°C, stirring was performed for a further 1 hour. The liquid was heated at 70-75°C, after which stirring was performed at the temperature for 1 hour to bring hydrolytic condensation reaction of methyltrimethoxysilane to completion.

The liquid obtained from hydrolytic condensation reaction of methyltrimethoxysilane in the water dispersion of silicone rubber spherical particles was dewatered by a pressure filter to a water content of about 30%. The dewatered product was transferred to a glass flask of 2 L volume equipped with an agitator having an anchor shape impeller. 1,000 g of water was added and stirring was performed for 30 minutes, followed by dewatering by a pressure filter. The dewatered product was transferred again to a glass flask of 2 L volume equipped with an agitator having an anchor shape impeller. 1,000 g of water was added and stirring was performed for 30 minutes, followed by dewatering by a pressure filter. The dewatered product was dried on a hot air fluidizing dryer at a temperature of 105°C. The dry product was disintegrated on a jet mill, obtaining flowing particles.

The particles were observed under an electron microscope, finding that silicone rubber spherical particles were covered over their entire surface with polymethylsilsesquioxane of granular shape (hydrolytic condensate of methyltrimethoxysilane). The silicone particles had a volume average particle size of 5 µm.

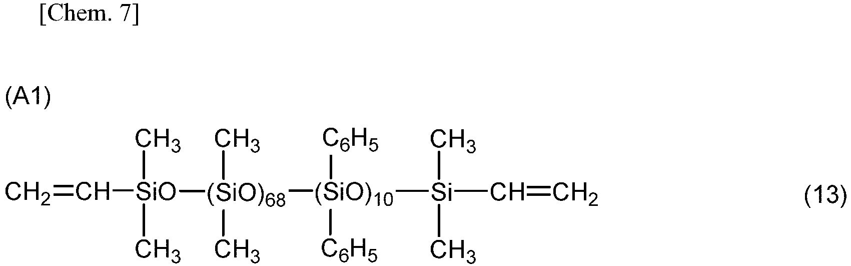

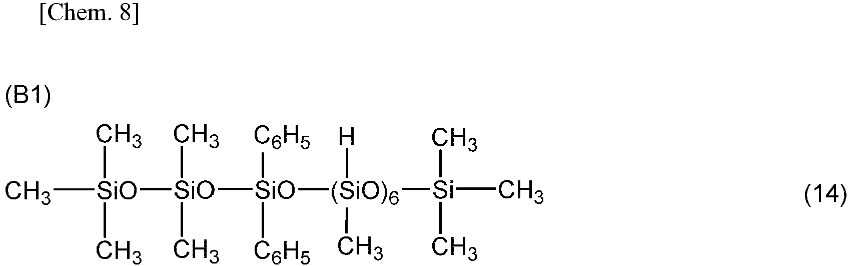

### [Example 2]

A glass beaker of 1 L volume was charged with 190 g of methylphenylvinylpolysiloxane (A2) having a kinematic viscosity of 850 mm²/s (and having a phenyl group content of 21.7 mol% based on silicon-bonded monovalent hydrocarbon groups exclusive of vinyl groups), represented by the average formula (15) shown below, and 10 g (to provide 1.21 SiH groups per vinyl group) of methylhydrogenpolysiloxane (B1) having a kinematic viscosity of 9 mm²/s (and having a phenyl group content of 14.3 mol% based on silicon-bonded monovalent hydrocarbon groups), represented by the average formula (14), which were stirred and dissolved using a homo-mixer at 2,000 rpm. Then, 1.2 g of polyoxyethylene lauryl ether (molar amount of ethylene oxide added = 9 mol) and 38 g of water were added to the solution, which was stirred using the homo-mixer at 6,000 rpm, with conversion to oil-in-water type and a viscosity buildup being acknowledged. Stirring was continued for a further 15 minutes. With stirring at 2,000 rpm, 758 g of water was added, obtaining a uniform white emulsion. The emulsion was transferred to a glass flask of 1 L volume equipped with an agitator having an anchor shape impeller and conditioned at 15-20°C. With stirring, a mixed/dissolved mixture of 0.7 g of an isododecane solution of platinum/vinyl-containing disiloxane complex (platinum content 0.5%) and 1.0 g of polyoxyethylene lauryl ether (molar amount of ethylene oxide added = 9 mol), and 1.4 g of polyoxyethylene lauryl ether (molar amount of ethylene oxide added = 4 mol) were added to the emulsion, which was stirred at the temperature for 1 hour. Thereafter, the mixture was heated at 30-40°C and stirred at the temperature for 5 hours, obtaining a water dispersion of silicone rubber particles.

The silicone rubber particles were observed under an optical microscope, finding a spherical shape and a volume average particle size of 5 µm (the measurement of volume average particle size is the same, hereinafter).

Using the water dispersion of silicone rubber spherical particles, the particles in which silicone rubber spherical particles were covered on their surface with polymethylsilsesquioxane were prepared as in Example 1.

The particles were observed under an electron microscope, finding that silicone rubber spherical particles were covered over their entire surface with polymethylsilsesquioxane of granular shape (hydrolytic condensate of methyltrimethoxysilane). The silicone particles had a volume average particle size of 5 µm.

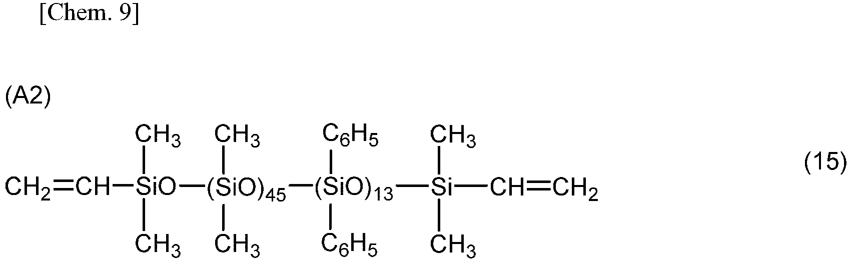

### [Example 3]

A glass beaker of 1 L volume was charged with 192 g of methylphenylvinylpolysiloxane (A3) having a kinematic viscosity of 1,020 mm²/s (and having a phenyl group content of 20.0 mol% based on silicon-bonded monovalent hydrocarbon groups exclusive of vinyl groups), represented by the average formula (16) shown below, and 8 g (to provide 1.25 SiH groups per vinyl group) of methylhydrogenpolysiloxane (B1) having a kinematic viscosity of 9 mm²/s (and having a phenyl group content of 14.3 mol% based on silicon-bonded monovalent hydrocarbon groups), represented by the average formula (14), which were stirred and dissolved using a homo-mixer at 2,000 rpm. Then, 1.2 g of polyoxyethylene lauryl ether (molar amount of ethylene oxide added = 9 mol) and 35 g of water were added to the solution, which was stirred using the homo-mixer at 6,000 rpm, with conversion to oil-in-water type and a viscosity buildup being acknowledged. Stirring was continued for a further 15 minutes. With stirring at 2,000 rpm, 761 g of water was added, obtaining a uniform white emulsion. The emulsion was transferred to a glass flask of 1 L volume equipped with an agitator having an anchor shape impeller and conditioned at 15-20°C. With stirring, a mixed/dissolved mixture of 0.7 g of an isododecane solution of platinum/vinyl-containing disiloxane complex (platinum content 0.5%) and 1.0 g of polyoxyethylene lauryl ether (molar amount of ethylene oxide added = 9 mol), and 1.4 g of polyoxyethylene lauryl ether (molar amount of ethylene oxide added = 4 mol) were added to the emulsion, which was stirred at the temperature for 1 hour. Thereafter, the mixture was heated at 30-40°C and stirred at the temperature for 5 hours, obtaining a water dispersion of silicone rubber particles.

The silicone rubber particles were observed under an optical microscope, finding a spherical shape and a volume average particle size of 5 µm.

Using the water dispersion of silicone rubber spherical particles, the particles in which silicone rubber spherical particles were covered on their surface with polymethylsilsesquioxane were prepared as in Example 1.

The particles were observed under an electron microscope, finding that silicone rubber spherical particles were covered over their entire surface with polymethylsilsesquioxane of granular shape (hydrolytic condensate of methyltrimethoxysilane). The silicone particles had a volume average particle size of 6 µm.

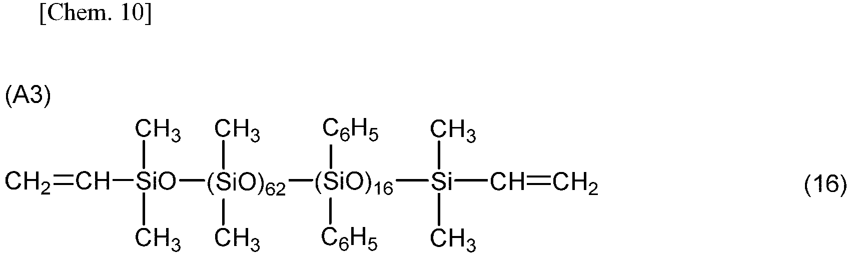

### [Comparative Example 1]

A glass beaker of 1 L volume was charged with 187 g of methylvinylpolysiloxane (a1) having a kinematic viscosity of 270 mm²/s (and having a phenyl group content of 0 mol% based on silicon-bonded monovalent hydrocarbon groups exclusive of vinyl groups), represented by the average formula (17) shown below, and 13 g (to provide 1.19 SiH groups per vinyl group) of methylhydrogenpolysiloxane (b1) having a kinematic viscosity of 30 mm²/s (and having a phenyl group content of 0 mol% based on silicon-bonded monovalent hydrocarbon groups), represented by the average formula (18) shown below, which were stirred and dissolved using a homo-mixer at 2,000 rpm. Then, 1.2 g of polyoxyethylene lauryl ether (molar amount of ethylene oxide added = 9 mol) and 22 g of water were added to the solution, which was stirred using the homo-mixer at 6,000 rpm, with conversion to oil-in-water type and a viscosity buildup being acknowledged. Stirring was continued for a further 15 minutes. With stirring at 2,000 rpm, 774 g of water was added, obtaining a uniform white emulsion. The emulsion was transferred to a glass flask of 1 L volume equipped with an agitator having an anchor shape impeller and conditioned at 15-20°C. With stirring, a mixed/dissolved mixture of 0.5 g of an isododecane solution of platinum/vinyl-containing disiloxane complex (platinum content 0.5%) and 0.6 g of polyoxyethylene lauryl ether (molar amount of ethylene oxide added = 9 mol) was added to the emulsion, which was stirred at the temperature for 5 hours, obtaining a water dispersion of silicone rubber particles.

The silicone rubber particles were observed under an optical microscope, finding a spherical shape and a volume average particle size of 5 µm.

Using the water dispersion of silicone rubber spherical particles, the particles in which silicone rubber spherical particles were covered on their surface with polymethylsilsesquioxane were prepared as in Example 1.

The particles were observed under an electron microscope, finding that silicone rubber spherical particles were covered over their entire surface with polymethylsilsesquioxane of granular shape (hydrolytic condensate of methyltrimethoxysilane). The silicone particles had a volume average particle size of 5 µm.

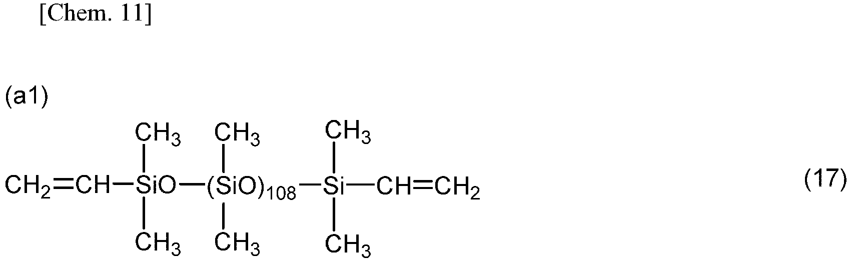

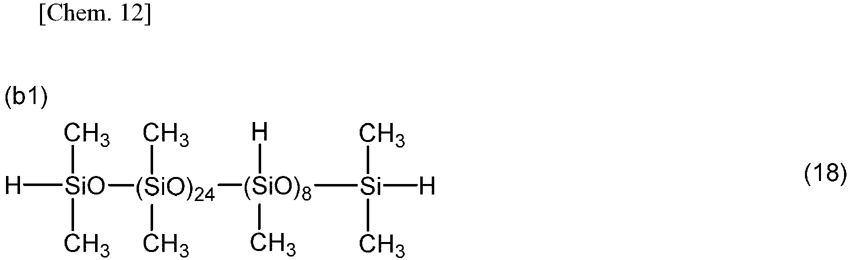

### [Comparative Example 2]

A glass beaker of 1 L volume was charged with 183 g of methylphenylvinylpolysiloxane (A4) having a kinematic viscosity of 1,100 mm²/s (and having a phenyl group content of 30.0 mol% based on silicon-bonded monovalent hydrocarbon groups exclusive of vinyl groups), represented by the average formula (19) shown below, and 17 g (to provide 1.18 SiH groups per vinyl group) of methylhydrogenpolysiloxane (B1) having a kinematic viscosity of 9 mm²/s (and having a phenyl group content of 14.3 mol% based on silicon-bonded monovalent hydrocarbon groups), represented by the average formula (14), which were stirred and dissolved using a homo-mixer at 2,000 rpm. Then, 1.2 g of polyoxyethylene lauryl ether (molar amount of ethylene oxide added = 9 mol) and 41 g of water were added to the solution, which was stirred using the homo-mixer at 6,000 rpm, with conversion to oil-in-water type and a viscosity buildup being acknowledged. Stirring was continued for a further 15 minutes. With stirring at 2,000 rpm, 755 g of water was added, obtaining a uniform white emulsion. The emulsion was transferred to a glass flask of 1 L volume equipped with an agitator having an anchor shape impeller and conditioned at 15-20°C. With stirring, a mixed/dissolved mixture of 0.7 g of an isododecane solution of platinum/vinyl-containing disiloxane complex (platinum content 0.5%) and 1.0 g of polyoxyethylene lauryl ether (molar amount of ethylene oxide added = 9 mol), and 1.4 g of polyoxyethylene lauryl ether (molar amount of ethylene oxide added = 4 mol) were added to the emulsion, which was stirred at the temperature for 1 hour. Thereafter, the mixture was heated at 30-40°C and stirred at the temperature for 5 hours, obtaining a water dispersion of silicone rubber particles.

The silicone rubber particles were observed under an optical microscope, finding a spherical shape and a volume average particle size of 5 µm.

Using the water dispersion of silicone rubber spherical particles, the particles in which silicone rubber spherical particles were covered on their surface with polymethylsilsesquioxane were prepared as in Example 1.

The particles were observed under an electron microscope, finding that silicone rubber spherical particles were covered over their entire surface with polymethylsilsesquioxane of granular shape (hydrolytic condensate of methyltrimethoxysilane). The silicone particles had a volume average particle size of 5 µm.

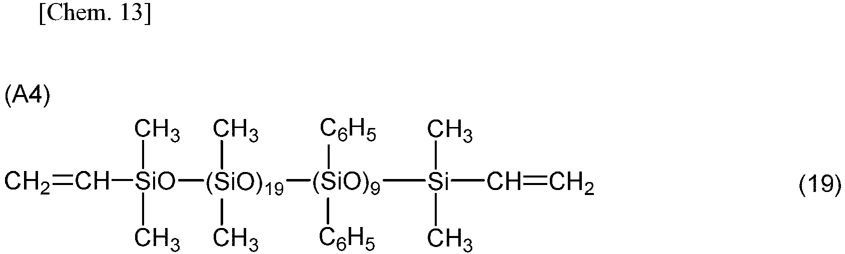

### [Measurement of hardness of silicone rubber particles]

A mixture was obtained by mixing the methylphenylvinylpolysiloxane or methylvinylpolysiloxane, the methylphenylhydrogenpolysiloxane or methylhydrogenpolysiloxane, and the isododecane solution of platinum/vinyl-containing disiloxane complex (platinum content 0.5%) used in Examples and Comparative Examples in the amounts shown in Examples and Comparative Examples and cast into an aluminum dish so as to reach a thickness of 10 mm. The mixture was allowed to stand at 25°C for 24 hours, and heated in a thermostatic tank at 50°C for 1 hour, obtaining a tack-free silicone rubber. The hardness of the silicone rubber was measured by Type A Durometer according to JIS K-6253. The results are shown in Table 1.

### [Measurement of refractive index of silicone rubber particles]

A mixture was obtained by mixing the methylphenylvinylpolysiloxane or methylvinylpolysiloxane, the methylphenylhydrogenpolysiloxane or methylhydrogenpolysiloxane, and the isododecane solution of platinum/vinyl-containing disiloxane complex (platinum content 0.5%) used in Examples and Comparative Examples in the amounts shown in Examples and Comparative Examples and placed in a sample stage of a digital refractometer. The sample was allowed to stand at 25°C for 24 hours, after which the cured rubber was measured for refractive index.

**[Table 1]**

| | Example 1 | Example 2 | Example 3 | Comparative Example 1 | Comparative Example 2 |
|---|---|---|---|---|---|
| Starting siloxanes to silicone rubber particles | (A1) | (A2) | (A3) | (a1) | (A4) |
| | (B1) | (B1) | (B1) | (b1) | (B1) |
| Hardness (Durometer A) of silicone rubber particles | 34 | 34 | 27 | 34 | 44 |
| Refractive index of silicone rubber particles | 1.45 | 1.49 | 1.49 | 1.41 | 1.52 |

### [Measurement of oil absorption of silicone particles]

The silicone particles obtained in Examples and Comparative Examples were measured for oil absorption per 100 parts by weight of silicone particles, using the oils used in the cosmetic application with reference to JIS K 5101-13-1, Section 13: oil absorption, Chapter 1: purified linseed oil method. The results are shown in Table 2 and plotted in FIG. 1.

**[Table 2]**

| Unit: g/100 g | Example 1 | Example 2 | Example 3 | Comparative Example 1 | Comparative Example 2 |
|---|---|---|---|---|---|
| KF-96A-6cs (*1) | 156 | 98 | 101 | 153 | 47 |
| KF-56A (*2) | 272 | 201 | 206 | 112 | 140 |
| Triethylhexanoin | 195 | 157 | 157 | 68 | 97 |
| Ethylhexyl methoxycinnamate | 128 | 114 | 117 | 46 | 76 |
| Squalane | 86 | 68 | 72 | 55 | 37 |

| | | | | | |
|---|---|---|---|---|---|
| (*1) dimethicone (INCI) having a kinematic viscosity of 6 mm²/s at 25°C by Shin-Etsu Chemical Co., Ltd. (*2) diphenylsiloxyphenyltrimethicone (INCI) by Shin-Etsu Chemical Co., Ltd. | | | | | |

As seen from the results of Table 2 and FIG. 1, Examples 1 to 3 have a greater oil absorption for each oil than Comparative Examples 1 and 2. In particular, Example 1 has a greater oil absorption for each oil than Examples 2 and 3.

### [Evaluation of utility of silicone particles]

### [Examples 4 to 6 & Comparative Examples 3 to 6: Water-in-oil cream]

A sun-screen cosmetic composition of the formulation shown below was prepared by the following method and evaluated for the following properties.

### (1) Evaluation of feeling on use

The sun-screen cosmetic composition of liquid emulsified form having formulated therein the silicone particles prepared in the above Examples and Comparative Examples was evaluated for feeling-on-use (spread and skin penetration) upon application and feeling-on-use (nonstick, gradation, smoothness) after application. The feeling-on-use was evaluated by a panel of 10 professional members. Evaluation was made according to the criterion shown in Table 3 and the result is an average of ratings of 10 panel members and rated according to the following judgment criterion. The results are also shown in Table 4.

**[Table 3]**

| Point | Evaluation criterion of feeling-on-use |
|---|---|
| 5 | Excellent |
| 4 | Good |
| 3 | Mediocre |
| 2 | Rather poor |
| 1 | Poor |

### (2) Judgment criterion of feeling-on-use

| | |
|---|---|
| ⊚: | 4.0 ≤ average point |
| ○: | 3.0 ≤ average point < 4.0 |
| Δ: | 2.0 ≤ average point < 3.0 |
| ×: | average point < 2.0 |

### [Examples 4 to 6 & Comparative Examples 3 to 6: Water-in-oil cream]

| Ingredients | % |
|---|---|
| 1. KSG-210 (*1) | 3.5 |
| 2. KSG-18A (*2) | 3.0 |
| 3. KF-6038 (*3) | 0.5 |
| 4. KF-56A (*4) | 5.0 |
| 5. Ethylhexyl methoxycinnamate | 7.5 |
| 6. KF-995 (*5) | 6.5 |
| 7. Silicone particles (shown in Table 4) | 5.0 |
| 8. BG | 5.5 |
| 9. Na citrate | 0.2 |
| 10. Na chloride | 0.5 |
| 11. Water | 62.8 |
| Total | 100.0 |

| | |
|---|---|
| (*1) mixture of cyclopentasiloxane (INCI) 80% + dimethicone/(PEG-10/15) crosspolymer (INCI) 20%, by Shin-Etsu Chemical Co., Ltd. (*2) mixture of diphenylsiloxyphenyltrimethicone (INCI) 85% + dimethicone/phenylvinyldimethicone crosspolymer (INCI) 15%, by Shin-Etsu Chemical Co., Ltd. (*3) lauryl PEG-9 polydimethylsiloxyethyl dimethicone (INCI) by Shin-Etsu Chemical Co., Ltd. (*4) diphenylsiloxyphenyltrimethicone (INCI) by Shin-Etsu Chemical Co., Ltd. (*5) cyclopentasiloxane (INCI) by Shin-Etsu Chemical Co., Ltd. (*6) vinyl dimethicone/methicone silsesquioxane crosspolymer (INCI) by Shin-Etsu Chemical Co., Ltd. to absorb 38 parts by weight of ethylhexyl methoxycinnamate per 100 parts by weight of silicone particles (*7) diphenyl dimethicone/vinyldiphenyl dimethicone/silsesquioxane crosspolymer (INCI) by Shin-Etsu Chemical Co., Ltd. to absorb 70 parts by weight of ethylhexyl methoxycinnamate per 100 parts by weight of silicone particles | |

### (Preparation method of Examples 4 to 6 and Comparative Examples 3 to 6)

The water-in-oil cream was prepared by the steps of:
A: mixing ingredients 1 to 7 to form an oily gel composition,
B: mixing ingredients 8 to 11,
C: adding the mixture of step B to the composition of step A and uniformly mixing, and
D: defoaming the mixture of step C and filling a container with the mixture.

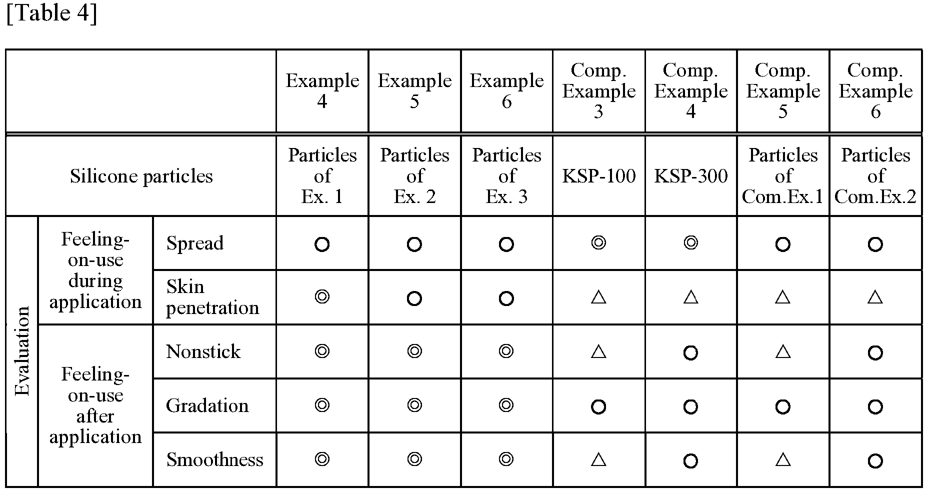

As is evident from the results of Table 4, Examples 1 to 3 have higher oil absorption than Comparative Examples. Since the silicone rubber spherical particles had a refractive index of 1.43 to 1.51 and a rubber hardness of 20 to 40, there were obtained cosmetic compositions (Examples 4 to 6) which were favorably evaluated in terms of skin penetration during application and feeling-on-use after application, although the spread during application was slightly heavy. Among others, Example 4 showed better results in terms of nonstick, gradation and smoothness after application.

### [Example 7: Water-in-oil cream]

| Formulation | % |
|---|---|
| 1. KSG-310 (*1) | 3.0 |
| 2. KSG-44 (*2) | 1.0 |
| 3. KF-6048 (*3) | 0.2 |
| 4. Squalane | 10.8 |
| 5. Silicone particles obtained in Example 3 | 1.0 |
| 6. BG | 8.0 |
| 7. Ethanol | 5.0 |
| 8. Mg sulfate | 0.2 |
| 9. Na chloride | 0.5 |
| 10. Water | balance |
| Total | 100.0 |

| | |
|---|---|
| (*1) mixture of mineral oil (INCI) 70% + PEG-15/lauryl dimethicone crosspolymer (INCI) 30%, by Shin-Etsu Chemical Co., Ltd. (*2) mixture of squalane (INCI) 70% + vinyl dimethicone/lauryl dimethicone crosspolymer (INCI) 30%, by Shin-Etsu Chemical Co., Ltd. (*3) cetyl PEG/PPG-10/1 dimethicone (INCI) by Shin-Etsu Chemical Co., Ltd. | |

### (Preparation method of Example 7)

A water-in-oil cream was prepared by the steps of:
A: mixing ingredients 1 to 5,
B: mixing ingredients 6 to 10,
C: adding the mixture of step B to the mixture of step A and uniformly mixing, and
D: defoaming the mixture of step C and filling a container with the mixture.

The water-in-oil cream (skin care cream) thus obtained was good in smoothness during application, had nonstick, light spreading or extending, and tight fit properties, and showed a naturally looking finish with proper settlement and less luster.

### [Example 8: Liquid emulsified foundation]

| Formulation | % |
|---|---|
| 1. KSG-710 (*1) | 4.0 |
| 2. KSG-15 (*2) | 2.0 |
| 3. KF-6105 (*3) | 3.0 |
| 4. KF-96A-6cs (*4) | 12.0 |
| 5. disteardimonium hectorite | 1.2 |
| 6. Silicone particles obtained in Example 1 | 3.0 |
| 7. KF-7312J (*5) | 5.0 |
| 8. Isotridecylisononanoate | 2.0 |
| 9. KF-6106 (*6) | 0.5 |
| 10. KTP-09W (*7) | proper |
| 11. KTP-09R (*7) | proper |
| 12. KTP-09Y (*7) | proper |
| 13. KTP-09B (*7) | proper |
| 14. Fragrance | proper |
| 15. Pentylene glycol | 5.0 |
| 16. Na citrate | 0.2 |
| 17. Na chloride | 0.5 |
| 18. Water | balance |
| Total | 100.0 |

| | |
|---|---|
| (*1) mixture of dimethicone (INCI) 75% + dimethicone/polyglycerin-3 crosspolymer (INCI) 25%, by Shin-Etsu Chemical Co., Ltd. (*2) mixture of cyclopentasiloxane (INCI) 93% + dimethicone/vinyl dimethicone crosspolymer (INCI) 7%, by Shin-Etsu Chemical Co., Ltd. (*3) lauryl polyglyceryl-3 polydimethylsiloxyethyl dimethicone (INCI) by Shin-Etsu Chemical Co., Ltd. (*4) dimethicone (INCI) having a kinematic viscosity of 6 mm²/s at 25°C by Shin-Etsu Chemical Co., Ltd. (*5) **solution** of cyclopentasiloxane (INCI) 50% + trimethylsiloxysilicic acid (labeling name, INCI: Trimethylsiloxysilicate) 50%, by Shin-Etsu Chemical Co., Ltd. (*6) polyglyceryl-3 polydimethylsiloxyethyl dimethicone (INCI) by Shin-Etsu Chemical Co., Ltd. (*7) coloring inorganic pigments treated with triethoxysilylethyl polydimethylsiloxyethylhexyl dimethicone (INCI), W: white, R: red, Y: yellow, B: black, by Shin-Etsu Chemical Co., Ltd. | |

### (Preparation method of Example 8)

A liquid emulsified foundation was prepared by the steps of:
A: mixing ingredients 1 to 7,
B: mixing ingredients 8 to 13,
C: adding the mixture of step B to the mixture of step A and uniformly mixing,
D: mixing ingredients 14 to 18,
E: adding the mixture of step D to the mixture of step C and uniformly mixing, and
F: defoaming the mixture of step E and filling a container with the mixture.

The liquid emulsified foundation thus obtained was good in smoothness during application and humidity, had nonstick, light spreading or extending, and tight fit properties, and showed a naturally looking finish with proper settlement and less luster.

### [Example 9: Powder foundation]

| Formulation | % |
|---|---|
| 1. Neopentylglycol diethylhexanoate | 4.0 |
| 2. Mineral oil | 2.0 |
| 3. KF-56A (*1) | 2.0 |
| 4. Dipentaerythrityl hexahydroxystearate/ hexastearate/hexarosinate | 0.2 |
| 5. Zinc stearate | 1.0 |
| 6. Silicone particles obtained in Example 2 | 5.0 |
| 7. Polymethylsilsesquioxane (*2) | 3.0 |
| 8. Boron nitride | 3.0 |
| 9. KF-99P-treated mica (*3) | 20.0 |
| 10. KF-99P-treated talc (*3) | balance |
| 11. Pigment grade titanium oxide (white) treated with AES-3083 (*4) | proper |
| 12. Iron oxide (red) treated with AES-3083 (*4) | proper |
| 13. Iron oxide (yellow) treated with AES-3083 (*4) | proper |
| 14. Iron oxide (black) treated with AES-3083 (*4) | proper |
| Total | 100.0 |

| | |
|---|---|
| (*1) diphenylsiloxyphenyltrimethicone (INCI) by Shin-Etsu Chemical Co., Ltd. (*2) polymethylsilsesquioxane (INCI) by Shin-Etsu Chemical Co., Ltd. (*3) treated with methicone (INCI), by Shin-Etsu Chemical Co., Ltd. (*4) treated with triethoxycaprylylsilane (INCI), by Shin-Etsu Chemical Co., Ltd. | |

### (Preparation method of Example 9)

A powder foundation was prepared by the steps of:
A: mixing ingredients 1 to 4,
B: mixing ingredients 5 to 14,
C: adding the mixture of step A to the mixture of step B and mixing, and
D: passing the mixture of step C through a screen and compacting in a metal dish in a mold.

The powder foundation thus obtained was good in smoothness and adhesion during application and had a long lasting finish.

### [Example 10: Water-in-oil concealer]

| Formulation | % |
|---|---|
| 1. KSG-210 (*1) | 3.0 |
| 2. KSG-15 (*2) | 5.0 |
| 3. KF-6028 (*3) | 2.0 |
| 4. KF-96L-2cs (*4) | balance |
| 5. Silicone particles obtained in Example 1 | 10.0 |
| 6. KP-545 (*5) | 3.0 |
| 7. Ethylhexyl palmitate | 2.0 |
| 8. KP-578 (*6) | 0.3 |
| 9. KTP-09W (*7) | proper |
| 10. KTP-09R (*7) | proper |
| 11. KTP-09Y (*7) | proper |
| 12. KTP-09B (*7) | proper |
| 13. BG | 5.0 |
| 14. Ethanol | 8.0 |
| 15. Na citrate | 0.2 |
| 16. Na chloride | 0.5 |
| 17. Water | 30.0 |
| Total | 100.0 |

| | |
|---|---|
| (*1) mixture of cyclopentasiloxane (INCI) 80% + dimethicone/PEG-10/15 crosspolymer (INCI) 20%, by Shin-Etsu Chemical Co., Ltd. (*2) mixture of cyclopentasiloxane (INCI) 93% + dimethicone/vinyl dimethicone crosspolymer (INCI) 7%, by Shin-Etsu Chemical Co., Ltd. (*3) PEG-9 polydimethylsiloxyethyl dimethicone (INCI) by Shin-Etsu Chemical Co., Ltd. (*4) dimethicone (INCI) having a kinematic viscosity of 2 mm²/s at 25°C by Shin-Etsu Chemical Co., Ltd. (*5) solution of cyclopentasiloxane (INCI) 70% + acrylate/dimethicone copolymer (INCI), by Shin-Etsu Chemical Co., Ltd. (*6) (acrylate/ethylhexyl acrylate/dimethicone methacrylate) copolymer (labeling name, INCI: Acrylates/Ethylhexyl Acrylate/Dimethicone Methacrylate Copolymer) by Shin-Etsu Chemical Co., Ltd. (*7) coloring inorganic pigments treated with triethoxysilylethyl polydimethylsiloxyethylhexyl dimethicone (INCI), W: white, R: red, Y: yellow, B: black, by Shin-Etsu Chemical Co., Ltd. | |

### (Preparation method of Example 10)

A water-in-oil concealer was prepared by the steps of:
A: mixing ingredients 1 to 6,
B: mixing ingredients 7 to 12 and roll milling,
C: adding the mixture of step B to the mixture of step A and uniformly mixing,
D: mixing ingredients 13 to 17,
E: adding the mixture of step D to the mixture of step C and uniformly mixing, and
F: defoaming the mixture of step E and filling a container with the mixture.

The water-in-oil concealer thus obtained was smooth and nonstick during application and showed a long lasting finish with tight adhesion and less luster.

### [Example 11: Lipstick]

| Formulation | % |
|---|---|
| 1. Polyethylene wax | 5.0 |
| 2. Ceresin | 2.0 |
| 3. Microcrystalline wax | 3.0 |
| 4. Candelilla wax | 1.0 |
| 5. Diisostearyl malate | 15.0 |
| 6. KP-561P (*1) | 2.0 |
| 7. Silicone particles obtained in Example 3 | 12.0 |
| 8. Sorbitan sesquiisostearate | 1.0 |
| 9. Polyglyceryl-2 triisostearate | 15.0 |
| 10. Glyceryl tri(caprylate/caprate) | balance |
| 11. Triethylhexanoin | 3.0 |
| 12. KP-578 (*2) | 0.5 |
| 13. Red #202 | proper |
| 14. Yellow #4 aluminum lake | proper |
| 15. Pigment grade titanium oxide (white) treated with KP-574 (*3) | proper |
| 16. Iron oxide (red) treated with KP-574 (*3) | proper |
| 17. Iron oxide (yellow) treated with KP-574 (*3) | proper |
| 18. Iron oxide (black) treated with KP-574 (*3) | proper |
| 19. Pearlescent agent of titanium oxide-coated mica | proper |
| Total | 100.0 |

| | |
|---|---|
| (*1) (acrylate/stearyl acrylate/dimethicone methacrylate) copolymer (labeling name, INCI: Acrylates/Stearyl Acrylate/Dimethicone Methacrylate Copolymer) by Shin-Etsu Chemical Co., Ltd. (*2) (acrylate/ethylhexyl acrylate/dimethicone methacrylate) copolymer (labeling name, INCI: Acrylates/Ethylhexyl Acrylate/Dimethicone Methacrylate Copolymer) by Shin-Etsu Chemical Co., Ltd. (*3) (acrylate/tridecyl acrylate/ triethoxysilylpropyl methacrylate/dimethicone methacrylate) copolymer (labeling name, INCI: Acrylates/Tridecyl Acrylate/Triethoxysilylpropyl Methacrylate/Dimethicone Methacrylate Copolymer) by Shin-Etsu Chemical Co., Ltd. | |

### (Preparation method of Example 11)

A lipstick was prepared by the steps of:
A: heating ingredients 1 to 10 for dissolution,
B: uniformly mixing ingredients 11 to 18 and roll milling,
C: adding the mixture of step B and ingredient 19 to the mixture of step A and uniformly mixing, and
D: defoaming the mixture of step C and filling a container with the mixture.

The lipstick thus obtained was smooth, nonstick, and lightly spreadable or extendable during application and showed a matte finish with tight adhesion, good settlement and less luster.

### [Example 12: Oil-in-water base cream]

| Formulation | % |
|---|---|
| 1. Water | balance |
| 2. Glycerin | 3.0 |
| 3. Microcrystalline wax | 3.0 |
| 4. Xanthan gum | 0.2 |
| 5. Pentylene glycol | 2.0 |
| 6. BG | 5.0 |
| 7. Sucrose coconut fatty acid | 0.2 |
| 8. Sorbitan stearate | 3.0 |
| 9. PEG-60 glyceryl isostearate | 0.5 |
| 10. Behenyl alcohol | 0.5 |
| 11. Ethylhexyl palmitate | 3.0 |
| 12. Silicone particles obtained in Example 2 | 3.0 |
| 13. Triethylhexanoin | 6.0 |
| 14. Polyhydroxystearic acid | 0.5 |
| 15. Microparticulate titanium oxide treated with metal soap | 8.0 |
| 16. Pigment grade titanium oxide (white) treated with metal soap | proper |
| 17. Iron oxide (red) treated with metal soap | proper |
| 18. Iron oxide (yellow) treated with metal soap | proper |
| 19. Iron oxide (black) treated with metal soap | proper |
| 20. Polysorbate 60 | 0.3 |
| 21. Hydroxyethyl acrylate/sodium acryloyldimethyltaurate copolymer | 0.6 |
| Total | 100.0 |

### (Preparation method of Example 12)

An oil-in-water base cream was prepared by the steps of:
A: uniformly mixing ingredients 1 to 6,
B: heating ingredients 7 to 11 for dissolution, adding ingredient 12 thereto, and uniformly mixing,
C: mixing ingredients 13 to 19 and roll milling the mixture,
D: adding the mixture of step C to the mixture of step B and uniformly mixing,
E: adding the mixture of step D to the mixture of step A both after heating, and uniformly mixing,
F: cooling the mixture of step E to normal temperature, adding ingredients 20 and 21, and uniformly mixing, and
G: defoaming the mixture of step F and filling a container with the mixture.

The oil-in-water base cream thus obtained was fully fresh, nonstick, and lightly spreadable or extendable during application and showed a matte finish with tight adhesion, good settlement and less luster.

### [Example 13: Aqueous gel]

| Formulation | % |
|---|---|
| 1. Silicone particles obtained in Example 2 | 5.0 |
| 2. KF-6100 (*1) | 0.5 |
| 3. Ethanol | 3.0 |
| 4. BG | 4.0 |
| 5. Glycerin | 2.0 |
| 6. Ammonium acryloyldimethyltaurate/VP copolymer | 0.2 |
| 7. Xanthan gum | 0.2 |
| 8. Arginine | 0.5 |
| 9. Phenoxyethanol | 0.3 |
| 10. Water | balance |
| Total | 100.0 |

| | |
|---|---|
| (*1) polyglyceryl-3 disiloxane dimethicone (INCI), by Shin-Etsu Chemical Co., Ltd. | |

### (Preparation method of Example 13)

An aqueous gel was prepared by the steps of:
A: uniformly mixing ingredients 1 to 3,
B: uniformly mixing ingredients 4 to 10,
C: adding the mixture of step A to the mixture of step B and uniformly mixing, and
D: defoaming the mixture of step C and filling a container with the mixture.

The aqueous gel thus obtained was fully fresh, nonstick, and lightly spreadable or extendable during application and showed a matte finish with tight adhesion, good settlement and less luster.

### [Example 14: Oily gel]

| Formulation | % |
|---|---|
| 1. Silicone particles obtained in Example 1 | 10.0 |
| 2. KSG-16 (*1) | 20.0 |
| 3. KSG-15 (*2) | 30.0 |
| 4. KF-56A (*3) | 5.0 |
| 5. KF-995 (*4) | balance |
| Total | 100.0 |

| | |
|---|---|
| (*1) mixture of dimethicone (INCI) 75% + dimethicone/vinyl dimethicone crosspolymer (INCI) 25%, by Shin-Etsu Chemical Co., Ltd. (*2) mixture of cyclopentasiloxane (INCI) 93% + dimethicone/vinyl dimethicone crosspolymer (INCI) 7%, by Shin-Etsu Chemical Co., Ltd. (*3) diphenylsiloxyphenyl trimethicone (INCI) by Shin-Etsu Chemical Co., Ltd. (*4) cyclopentasiloxane (INCI) by Shin-Etsu Chemical Co., Ltd. | |

### (Preparation method of Example 14)

An oily gel was prepared by the steps of:
A: uniformly mixing ingredients 1 to 5, and
B: defoaming the mixture of step A and filling a container with the mixture.

The oily gel thus obtained was smooth, nonstick, and lightly spreadable or extendable during application and showed a matte finish with tight adhesion, good settlement and less luster.

### [Example 15: Oily solid foundation]

| Formulation | % |
|---|---|
| 1. Synthetic wax | 4.0 |
| 2. Carnauba wax | 2.0 |
| 3. Shea butter | 0.5 |
| 4. Glyceryl tri(caprylate/caprate) | 3.0 |
| 5. KF-56A (*1) | 5.0 |
| 6. Ethylhexyl methoxycinnamate | 7.0 |
| 7. Bis-ethylhexyloxyphenol methoxyphenyl triazine | 0.5 |
| 8. KF-96L-2cs (*2) | 5.0 |
| 9. Isotridecyl isononanoate | balance |
| 10. Silicone particles obtained in Example 2 | 4.0 |
| 11. KMP-591 (*3) | 1.0 |
| 12. Cetyl ethylhexanoate | 3.0 |
| 13. KF-6115 (*4) | 1.0 |
| 14. Microparticulate titanium oxide treated with KF-99P (*5) | 7.0 |
| 15. KTP-09W (*6) | proper |
| 16. KTP-09R (*6) | proper |
| 17. KTP-09Y (*6) | proper |
| 18. KTP-09B (*6) | proper |
| Total | 100.0 |

| | |
|---|---|
| (*1) diphenylsiloxyphenyltrimethicone (INCI) by Shin-Etsu Chemical Co., Ltd. (*2) dimethicone (INCI) having a kinematic viscosity of 2 mm²/s at 25°C by Shin-Etsu Chemical Co., Ltd. (*3) polymethylsilsesquioxane (INCI) by Shin-Etsu Chemical Co., Ltd. (*4) laurylpolyglyceryl-3 polydimethylsiloxyethyl dimethicone (INCI) by Shin-Etsu Chemical Co., Ltd. (*5) treated with methicone (INCI) by Shin-Etsu Chemical Co., Ltd. (*6) coloring inorganic pigments treated with triethoxysilylethyl polydimethylsiloxyethylhexyl dimethicone (INCI), W: white, R: red, Y: yellow, B: black, by Shin-Etsu Chemical Co., Ltd. | |

### (Preparation method of Example 15)

An oily solid foundation was prepared by the steps of:
A: heating ingredients 1 to 9 for dissolution,
B: uniformly mixing ingredients 12 to 18 and roll milling the mixture,
C: adding the mixture of step B and ingredients 10 and 11 to the heated mixture of step A and uniformly mixing, and
D: defoaming the mixture of step C, filling a container with the heated mixture, and cooling to room temperature.

The oily solid foundation thus obtained showed light spread or extension and good skin settlement, and formed a tight cosmetic film with a moist feeling and less luster, which was long lasting and excellent.

### [Example 16: Oily foundation]

| Formulation | % |
|---|---|
| 1. KSG-42A (*1) | 10.0 |
| 2. Silicone particles obtained in Example 2 | 6.0 |
| 3. KF-6104 (*2) | 4.0 |
| 4. Disteardimonium hectorite | 1.5 |
| 5. Silica silylate | 1.0 |
| 6. TSPL-30-ID (*3) | 2.0 |
| 7. Dimethicone (*4) | 5.0 |
| 8. Ethanol | 8.0 |
| 9. Isododecane | balance |
| 10. Isotridecyl isononanoate | 10.0 |
| 11. KF-578 (*5) | 0.5 |
| 12. Microparticulate titanium oxide treated with KF-9901 (*6) | 8.0 |
| 13. Microparticulate zinc oxide treated with KF-9901 (*6) | 5.0 |
| 14. Pigment grade titanium oxide (white) treated with AES-3083 (*7) | proper |
| 15. Iron oxide (red) treated with AES-3083 (*7) | proper |
| 16. Iron oxide (yellow) treated with AES-3083 (*7) | proper |
| 17. Iron oxide (black) treated with AES-3083 (*7) | proper |
| Total | 100.0 |

| | |
|---|---|
| (*1) mixture of isododecane (INCI) 80% + vinyl dimethicone/lauryl dimethicone crosspolymer (INCI) 20%, by Shin-Etsu Chemical Co., Ltd. (*2) polyglyceryl-3 polydimethylsiloxyethyl dimethicone (INCI) by Shin-Etsu Chemical Co., Ltd. (*3) solution of isododecane (INCI) 70% + pullulan trimethylsiloxysilylpropylcarbamate (labeling name, INCI: Trimethylsiloxysilylpropylcarbamoyl Pullulan) 30%, by Shin-Etsu Chemical Co., Ltd. (*4) dimethicone (INCI) having a kinematic viscosity of 6 mm²/s at 25°C by Shin-Etsu Chemical Co., Ltd. (*5) acrylate/ethylhexyl acrylate/dimethicone methacrylate copolymer (labeling name, INCI: Acrylates/Ethylhexyl Acrylate/Dimethicone Methacrylate Copolymer) by Shin-Etsu Chemical Co., Ltd. (*6) hydrogendimethicone (INCI) by Shin-Etsu Chemical Co., Ltd. (*7) triethoxycaprylylsilane (INCI) by Shin-Etsu Chemical Co., Ltd. | |

### (Preparation method of Example 16)

An oily foundation was prepared by the steps of:
A: uniformly mixing ingredients 1 to 9,
B: uniformly mixing ingredients 10 to 17 and roll milling the mixture,
C: adding the mixture of step B to the mixture of step A and uniformly mixing, and
D: defoaming the mixture of step C and filling a container with the mixture.

The oily foundation thus obtained showed light spread or extension and good skin settlement, and formed a tight cosmetic film with a moist feeling and less luster, which was long lasting and excellent.

### [Example 17: Oily mascara]

| Formulation | % |
|---|---|
| 1. Paraffin wax | 20.0 |
| 2. Microcrystalline wax | 8.0 |
| 3. Polyethylene wax | 3.0 |
| 4. Inulin stearate | 1.0 |
| 5. Disteardimonium hectorite | 2.0 |
| 6. KF-6028 (*1) | 1.0 |
| 7. NBN-30-ID (*2) | 3.0 |
| 8. Hydrogenated polyisobutene | balance |
| 9. Silicone particles obtained in Example 3 | 2.0 |
| 10. Neopentylglycol dicaprate | 5.0 |
| 11. KF-6115 (*3) | 1.0 |
| 12. KTP-09W (*4) | proper |
| 13. KTP-09B (*4) | proper |
| Total | 100.0 |

| | |
|---|---|
| (*1) PEG-9 polydimethylsiloxyethyl dimethicone (INCI) by Shin-Etsu Chemical Co., Ltd. (*2) solution of isododecane (INCI) 70% + norbornene/tris(trimethylsiloxy)silylnorbornene copolymer (INCI) 30%, by Shin-Etsu Chemical Co., Ltd. (*3) lauryl polyglyceryl-3 polydimethylsiloxyethyl dimethicone (INCI) by Shin-Etsu Chemical Co., Ltd. (*4) coloring inorganic pigments treated with triethoxysilylethyl polydimethylsiloxyethylhexyl dimethicone (INCI), W: white, R: red, Y: yellow, B: black, by Shin-Etsu Chemical Co., Ltd. | |

### (Preparation method of Example 17)

An oily mascara was prepared by the steps of:
A: heating ingredients 1 to 8 for dissolution,
B: uniformly mixing ingredients 10 to 13 and roll milling the mixture,
C: adding the mixture of step B and ingredient 9 to the heated mixture of step A and uniformly mixing, and
D: defoaming the mixture of step C, cooling to room temperature, and filling a container with the mixture.

The oily mascara thus obtained showed was smooth, nonstick, and lightly spreadable or extensible upon application, tight adhesion and good settlement, and showed a less lustrous, non-oozing finish, which was long lasting and excellent.

### [Example 18: Loose powder]

| Formulation | % |
|---|---|
| 1. Silicone particles obtained in Example 2 | 10.0 |
| 2. Synthetic fluorophlogopite treated with KF-9901 (*1) | 25.0 |
| 3. Talc treated with KF-9901 (*1) | balance |
| 4. Lauroyl lysine | 5.0 |
| 5. Boron nitride | 3.0 |
| 6. Pearlescent agent of titanium oxide-coated mica | 3.0 |
| 7. Titanium oxide treated with AES-3083 (*2) | proper |
| 8. Red iron oxide treated with AES-3083 (*2) | proper |
| 9. Yellow iron oxide treated with AES-3083 (*2) | proper |
| 10. Black iron oxide treated with AES-3083 (*2) | proper |
| 11. Isononyl isononanoate | 2.0 |
| 12. Ethylhexylglycerin | 0.5 |
| Total | 100.0 |

| | |
|---|---|
| (*1) treated with hydrogen dimethicone (INCI), by Shin-Etsu Chemical Co., Ltd. (*2) treated with triethoxycaprylylsilane (INCI), by Shin-Etsu Chemical Co., Ltd. | |

### (Preparation method of Example 18)

A loose powder was prepared by the steps of:
A: uniformly mixing ingredients 1 to 10,
B: uniformly mixing ingredients 11 and 12,
C: adding the mixture of step B to the mixture of step A and uniformly mixing, and
D: passing the mixture of step C through a screen, and filling a container with the mixture.

The loose powder thus obtained was smooth upon application and showed a less lustrous finish, which was long lasting and excellent.

### [Example 19: Sun-screen milky lotion]

| Formulation | % |
|---|---|
| 1. KSG-270 (*1) | 3.0 |
| 2. KSG-18A (*2) | 3.0 |
| 3. KF-6048 (*3) | 2.0 |
| 4. KF-56A (*4) | 5.0 |
| 5. KF-4422 (*5) | balance |
| 6. Ethylhexyl methoxycinnamate | 5.0 |
| 7. Ethylhexyl salicylate | 2.0 |
| 8. Octocrylene | 1.0 |
| 9. Hexyl diethylaminohydroxybenzoylbenzoate | 2.0 |
| 10. Silicone particles obtained in Example 1 | 5.0 |
| 11. SPD-T5 (*6) | 10.0 |
| 12. SPD-Z5 (*7) | 10.0 |
| 13. BG | 3.0 |
| 14. Ethanol | 5.0 |
| 15. Na citrate | 0.2 |
| 16. Na chloride | 0.5 |
| 17. Water | 20.0 |
| Total | 100.0 |

| | |
|---|---|
| (*1) mixture of diphenylsiloxyphenyl trimethicone (INCI) 80% + dimethicone/PEG-10/15 crosspolymer (INCI) 20%, by Shin-Etsu Chemical Co., Ltd. (*2) mixture of diphenylsiloxyphenyl trimethicone (INCI) 85% + dimethicone/phenylvinyl dimethicone crosspolymer (INCI) 15%, by Shin-Etsu Chemical Co., Ltd. (*3) cetyl PEG/PPG-10/1 dimethicone (INCI) by Shin-Etsu Chemical Co., Ltd. (*4) diphenylsiloxyphenyl trimethicone (INCI) by Shin-Etsu Chemical Co., Ltd. (*5) ethyl trimethicone (INCI) by Shin-Etsu Chemical Co., Ltd. (*6) mixture of cyclopentasiloxane (INCI), titanium oxide (labeling name, (INCI: Titanium Dioxide), Al hydroxide (labeling name, INCI: Aluminum Hydroxide), and stearic acid (labeling name, INCI: Stearic Acid), by Shin-Etsu Chemical Co., Ltd. (*7) mixture of cyclopentasiloxane (INCI), zinc oxide (labeling name, INCI: Zinc Oxide), polyglyceryl-3 polydimethylsiloxyethyl dimethicone (INCI), and triethoxysilylethyl polydimethylsiloxyethylhexyl dimethicone (INCI), by Shin-Etsu Chemical Co., Ltd. | |

### (Preparation method of Example 19)

A sunscreen milky lotion was prepared by the steps of:
A: uniformly mixing ingredients 1 to 10,
B: uniformly mixing ingredients 13 to 17,
C: adding the mixture of step B to the mixture of step A and uniformly mixing,
D: adding ingredients 11 and 12 to the mixture of step C and uniformly mixing, and
E: defoaming the mixture of step D and filling a container with the mixture.

The sunscreen milky lotion thus obtained was smooth upon application and showed a less lustrous finish, which was long lasting and excellent.

## Claims

1. Cosmetic silicone particles comprising 100 parts by weight of silicone rubber spherical particles and 0.5 to 25 parts by weight of a polyorganosilsesquioxane covering the surface thereof, and having a volume average particle size of 0.5 to 100 µm,
100 parts by weight of the cosmetic silicone particles absorb at least 100 parts by weight of ethylhexyl methoxycinnamate,
the silicone rubber spherical particles have a refractive index of 1.43 to 1.51, and
the silicone rubber spherical particles are the addition reaction product of
(A) an organopolysiloxane containing at least two alkenyl groups per molecule, represented by the average compositional formula (1):
R¹ₐR²_{b}SiO_{(4-a-b)/2} (1)
wherein R¹ is each independently a substituted or unsubstituted, alkenyl-free, C₁-C₃₀ monovalent hydrocarbon group, 1 to 35 mol% of all R¹ being phenyl, R² is each independently a C₂-C₆ alkenyl group, a and b are numbers in the range: 0 < a < 3, 0 < b < 3, and 0.1 ≤ a+b < 3, and
(B) an organohydrogenpolysiloxane containing at least two silicon-bonded hydrogen atoms per molecule, represented by the average compositional formula (2):
R³_{c}H_{d}SiO_{(4-c-d)/2} (2)
wherein R³ is each independently a substituted or unsubstituted, alkenyl-free, C₁-C₃₀ monovalent hydrocarbon group, 1 to 35 mol% of all R³ being phenyl, c and d are numbers in the range: 0 < c < 3, 0 < d ≤ 3, and 0.1 ≤ c+d ≤ 3,
excluding the combination of (A) an organopolysiloxane containing only two alkenyl groups per molecule with (B) an organohydrogenpolysiloxane containing only two SiH groups per molecule.

2. The cosmetic silicone particles of claim 1 wherein 5 to 28 mol% of all R¹ in formula (1) is phenyl, and 5 to 25 mol% of all R³ in formula (2) is phenyl.

3. The cosmetic silicone particles of claim 1 wherein 100 parts by weight of the cosmetic silicone particles absorb at least 90 parts by weight of a silicone based oil selected from silicone oils and modified silicone oils and at least 50 parts by weight of a hydrocarbon based oil selected from sebum, hydrocarbon oils, and ester oils.

4. The cosmetic silicone particles of claim 1 wherein the silicone rubber spherical particles have a rubber hardness of 20 to 40 as measured by Type A Durometer according to JIS K6253.

5. A cosmetic composition comprising the cosmetic silicone particles of any one of claims 1 to 4.
